(19) Europäisches Patentamt
European Patent Office
Office européen des brevets

(11) Publication number: 0 141 779 A1

(12) EUROPEAN PATENT APPLICATION

(21) Application number: 84810471.7

(22) Date of filing: 28.09.84

(51) Int. Cl.[4]: C 12 N 15/00, C 12 P 21/02, C 07 K 7/10, A 61 K 37/02

(30) Priority: 03.10.83 US 538099

(43) Date of publication of application: 15.05.85 Bulletin 85/20

(84) Designated Contracting States: AT BE CH DE FR GB IT LI LU NL SE

(71) Applicant: SANDOZ AG, Lichtstrasse 35, CH-4002 Basel (CH)
(84) Designated Contracting States: BE CH FR GB IT LI LU NL SE

(71) Applicant: SANDOZ-PATENT-GMBH, Humboldtstrasse 3, D-7850 Lörrach (DE)
(84) Designated Contracting States: DE

(71) Applicant: SANDOZ-ERFINDUNGEN Verwaltungsgesellschaft m.b.H., Brunner Strasse 59, A-1235 Wien (AT)
(84) Designated Contracting States: AT

(72) Inventor: Clark, Steven C., 122 Johnson Road, Winchester, MA 01890 (US)
Inventor: Wrann, Michael, Brunnerbergstrasse 31/62, Berchtoldsdorf (AT)

(54) **T-cell growth factor.**

(57) The mRNA of the Gibbon ape T-Cell Growth Factor concentrated from a Cell line identified as UCD-MLA-144 lead to the production and cloning of its cDNA and the elucidation of its gene structure and that of the mature protein. Transfer vectors for production of the Gibbon ape T-Cell Growth Factor are disclosed along with useful intermediate expression products.

EP 0 141 779 A1

ACTORUM AG

## T-CELL GROWTH FACTOR OF THE GIBBON APE

The T-cell growth factor (TCGF), also known as Interleukin-2(IL-2), is a lymphokine produced by lectin- or antigen-activated T-cells in mammals.

The structure and expression of a precursor protein coded by a cloned gene constructed from human mRNA for TCGF was recently reported by Taniguchi et al., Nature, Vol. 302, 305-310 (March, 1983).

The present invention has among its objects the isolation of mRNA for the TCGF of the Gibbon ape (hereinafter also gTCGF), and its cloning and expression in recombinant vehicles to obtain a product exhibiting TCGF activity. Another object is to elucidate the sequence of gTCGF and of sequences for its production.

The following definitions are used throughout this text for purposes of clarification and/or shortening the necessary description:

Cloning vehicle, vector, transfer vector and the like - all refer to non-chromosonal DNA (double stranded unless otherwise indicated) and capable of replication (by reason of containing an intact replicon) when placed within a host, e.g. animal cells or a microorganism. However, the term cloning vehicle is often used when the purpose is primarily cloning (multiplication) and not expression, and the term transfer vector, when used, generally indicates a recombinant construct containing heterologous DNA (more particularly the whole or portion of a coding sequence whose expression is ultimately desired) and prepared in furtherance of the objectives of the invention. The term vector is used in the generic sense to cover plasmids, bacteriophages and other similarly useful vehicles, such as those used as starting materials herein, but is also used to refer to recombinant derivatives thereof. Hence, the terms may be used interchangeably and even overlap with regard to the above indicated more particular meanings usually desired to be emphasized in their use.

Transcription Control System - used herein with reference to the structure of recombinant vehicles such as in a vector, transfer vector and the like, to denote a double-stranded DNA region or regions comprising at least the DNA construction operatively necessary to control transcription of ribosomal translatable mRNA in a host. The term is also intended to encompass all of the elements which may be included in a particular system for purposes of controlling, regulating and enhancing transcription. Such elements include without limitation those well known as promoters and operators (together the promoter-operator system), catabolite gene activator protein (CAP) binding sites, ribosomal binding site sequences, N-protein utilisation sites (Nut sites) and eukaryotic transcriptional enhancer sequence. As will be understood, the elements minimally necessary to operatively effect transcription will depend upon various factors, particularly the host in which transcription is to be effected. For example, an operator may depend upon repressor protein produced by a regulator but the regulator may be located in the chromosone of the host in which the transfer vector is placed (e.g. by transformation) in which case such regulator will not by definition form a part of the transcription control system of the vector. Similarly, a sequence coding for a ribosomal binding site in order to produce translatable mRNA may be necessary only for certain hosts, such as bacterial hosts in general.

Recombinant Operon - a double-stranded DNA segment comprising a transcription control system and a sequence coding for gTCGF under its control.

Expression - is intended to be generic to the independent processes of transcription and translation, i.e. the production of mRNA and the production of a protein from the mRNA.

The above and other objects of the invention will be evident from the following description and the accompanying drawings in which:

Fig. 1 represents the overall cloning strategy by which isolated poly-(A) mRNA is enzymatically converted into duplex cDNA for recombinant integration into the plasmid pBR322 to produce a plurality of cloning vehicles pBR322-cDNA which are used to transform bacteria to obtain a library of clones which was screened to find the clone transformed with the cloning vehicle containing the cDNA of the gTCGF messenger, said desired cloning vehicle being designated pBR322-gTCGF.

Fig. 2 represents the strategy for obtaining by duplex DNA degradation segments of the gTCGF cDNA from pBR322-gTCGF for sequencing by the M13 dideoxy sequencing method. The five segments sequenced as a result of this procedure are also shown in Fig. 3 (see below).

Fig. 3 represents the strategy for obtaining by restriction endonuclease cleavage sections of the gTCGF cDNA duplex from pBR322-gTCGF for sequencing by the M13 dideoxy sequencing method. Fig. 3 also depicts the five degraded cDNA sections obtained by the method represented by Fig. 2.

Fig. 4 represents a strategy for obtaining by restriction endonuclease cleavage sections of the gTCGF cDNA duplexes from pBR322-gTCGF for sequencing by the Maxam-Gilbert method.

Fig. 5 shows the construction of a transfer vector pCVSVL-gTCGF for purposes of producing gTCGF in animal cells.

Fig. 6 shows the construction of a gTCGF coding segment useful for constructing a transfer vector pEVPL-gTCGF (Fig. 7, below) which is useful for producing gTCGF in bacteria.

Fig. 7 shows the construction of the transfer vector pEVPL-gTCGF derived from the plasmid pEVPL and capable of producing gTCGF in bacteria.

Fig. 8 shows the construction of the plasmid pEVPL.

Table A, below, depicts the amino acid sequence of gTCGF and the nucleotide sequence its cDNA segment which embodies the coding region for the mature protein, a signal peptide region, upstream (5') and downstream (3') untranslated regions coded for by the natural gene and an upstream artifact region which arose in the production of the cDNA. Amino acids are designated by conventional abbreviations and numbered at intervals above such designations. The nucleotides in the sequence are numbered below their designation in units of fifty with a dot placed at every interval of ten nucleotides beginning with the first nucleotide in the codon for 1-Met. Certain restriction sites within the sequence are also identified.

As a source material for the mRNA of the gTCGF we employed the Gibbon ape T-cell line UCD-MLA-144. Such cell line was obtained from a spontaneous lymphosarcoma of a Gibbon ape and overproduces gTCGF and its mRNA. The cell line and its origin have been described by Rubin et al., J. Immun., 127, No. 5, 1852-1855 (1981) and in the publications noted therein. The UCD-MLA-144 cell line has been deposited with the ATCC and assigned the accession number HB8370.

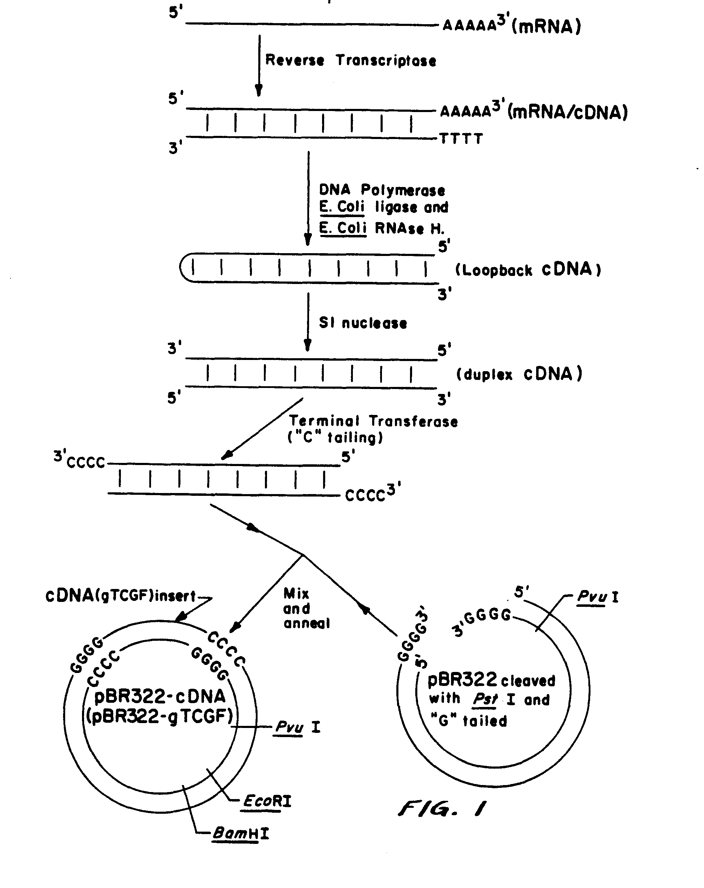

As exemplified hereinafter, the UCD-MLA-144 cell line may be cultured to multiply the cells and the multiplied cells induced to produce high levels of gTCGF and the mRNA thereof. The cells may then be lysed to free their cytoplasmic RNA content and the mRNA may then be isolated using an oligo dT cellulose column. The presence of desired mRNA for gTCGF in the collected mRNA mixture may then be ascertained using a standard *Xenopus laevis* translation system and suitable assay for TCGF activity. Having thus found significant levels of mRNA for gTCGF in the collected mRNA mixture, such mixture becomes useful for attempting the construction, isolation and identification of a gTCGF producing DNA duplex from the mRNA. This may be accomplished by first forming double stranded cDNA from the total mRNA in the mixture. As outlined in Fig. 1, treatment of the mRNA mixture with reverse transcriptase (to form mRNA/cDNA) followed by DNA polymerase, E. Coli ligase and E. Coli RNAsa H results in the production of double stranded hairpin loopback forms of cDNA and the hairpin loops may be successfully cleaved by S1-nuclease. The resulting duplex cDNA may be then cloned by integration of the cDNA into any of a variety of vectors, such as a plasmid, such as the plasmid pBR322 which may be used to form a plurality of cloning vehicles pBR322-cDNA, as shown in Fig. 1. The integration of the cDNA of unknown structure into the cloning vector may be facilitated by homopolymeric tailing of the ends of the cDNA and complementary homopolymeric tailing of the ends of the cloning vector which has been linearized by restriction cleavage, such tailing being desirably selected to reestablish restriction sites upon integration of the cDNA into the linearized vector. Thus, as shown in Fig. 1, the integration of the obtained cDNA into the plasmid pBR322 was effected by homopolymeric "C" tailing of the 3' ends of cDNA and complementary homopolymeric "G" tailing of the 3' ends of the plasmid pBR322 which had been cleaved with the restriction endonuclease Pst I to form cohesive termini. The "G" tailing of the 3' ends from such Pst I cleavage will lead to the reestablishment of Pst I cleavage sites in the transfer vector formed on integrating the "C" tailed cDNA into the "G" tailed linearized plasmid, thereby enabling the convenient removal of the inserted cDNA (gTCGF) segment as later desired from the cloning vehicles pBR322-cDNA.

The resulting mixture of cloning vehicles pBR322-cDNA are used to transform a suitable replication host, such as E. Coli (e.g. strain MC1061). Following standard procedures, the resulting transformants are dispersed on filters and cultured in the presence of antibiotic (to eliminate non-transformed bacteria) to obtain a library of individual clones (numbering about 7,500 in the exemplification hereinafter). Such library (following replica plating) can be employed to screen for the desired candidate clone(s) containing a cDNA insert with the gTCGF coding sequence.

The screening for the desired candidate clone(s) containing the gTCGF cDNA may be effected employing standard colony hybridization procedures along with a suitable identification means capable of locating the candidate clones. Such identification means may be $^{32}P$ labelled oligonucleotides of reasonable length and useful as probes by reason of each nucleotide therein being able to attach itself by hydrogen bonding to its complementary base pair nucleotide in the cDNA segment sought to be found, it being necessary that the structures be substantially fully complementary over their entire length. Since the structure of the gTCGF protein itself let alone the coding sequence therefor are unknown, it was decided to prepare and try oligonucleotide probes based on the possibility that portions of the gTCGF protein and its coding sequence might be the same as found by Taniguchi et al., supra, for human TCGF. Two such oligonucleotide probes each of 17 nucleotides and having the structures:

d(G-C-A-C-C-T-A-C-T-T-C-A-A-G-T-T-C), and

d(C-T-G-A-T-T-A-A-G-T-C-C-C-T-G-G-G)

were found in colony hybridization to both hydridize (bind) with one colony in a cluster of three colonies from which it was readily possible to ascertain the colony of candidate cells by separating the three colonies and finding (after culturing and lysing to isolate their contained cloning vehicles) that the cDNA inserts (liberated by Pst I restriction enzyme cleavage) from only one colony (its contained cloning vehicle hereinafter also "pBR322-gTCGF") hybridized to both probes in the Southern Blot hybridization procedure (this same candidate was also found on analysis of Pst I fragments to have a cDNA length indicating it to be the more likely candidate of the three clones). The cDNA insert of pBR322-gTCGF produced fragments of about 350 and 500 base pairs on restriction enzyme cleavage with the endonuclease Xba I (recognition sequence TCTAGA which is very rare in mammalian DNA) and thus increased confidence that the above described procedures provided for the successful isolation and cloning of the gTCGF sequence, by reason of the observation that the human TCGF gene embodies the same site at roughly a midpoint location.

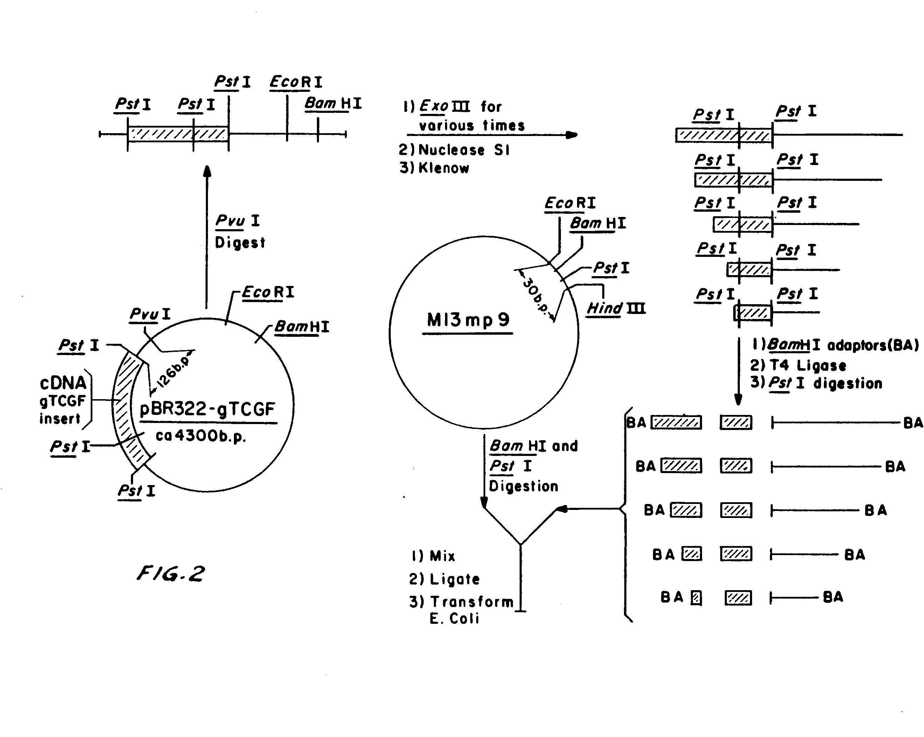

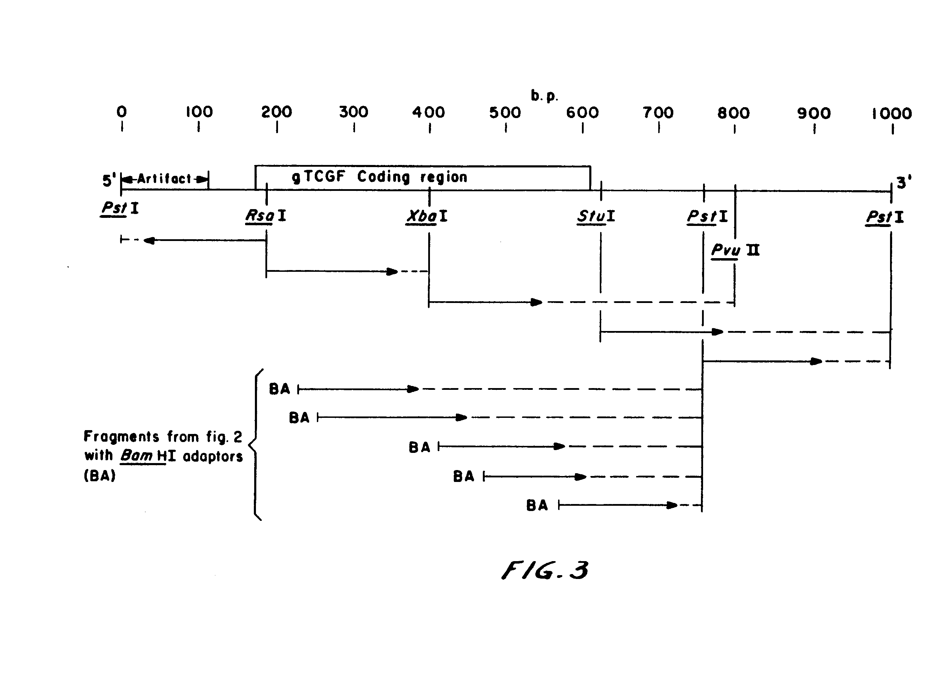

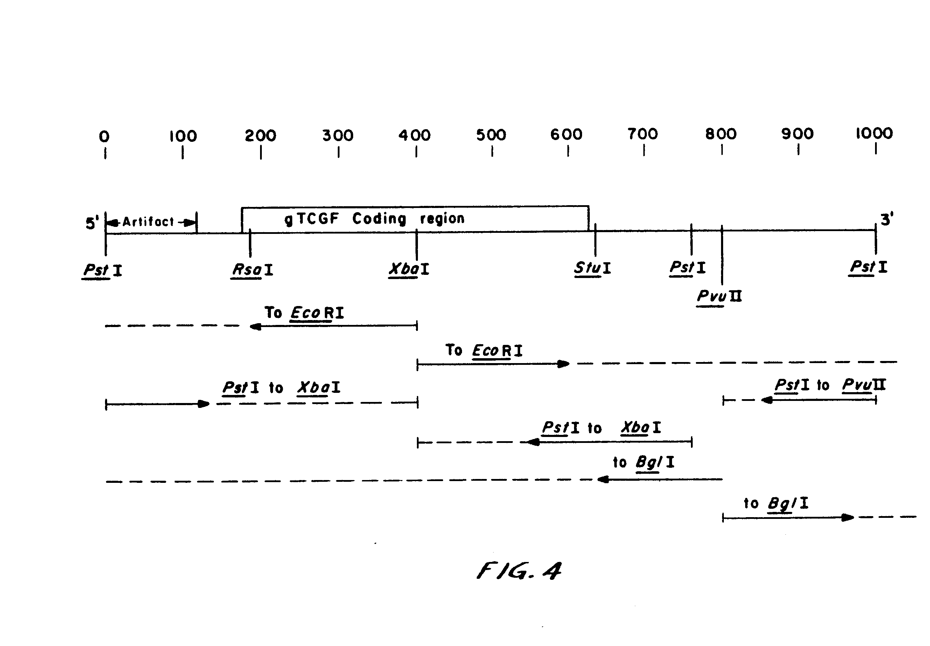

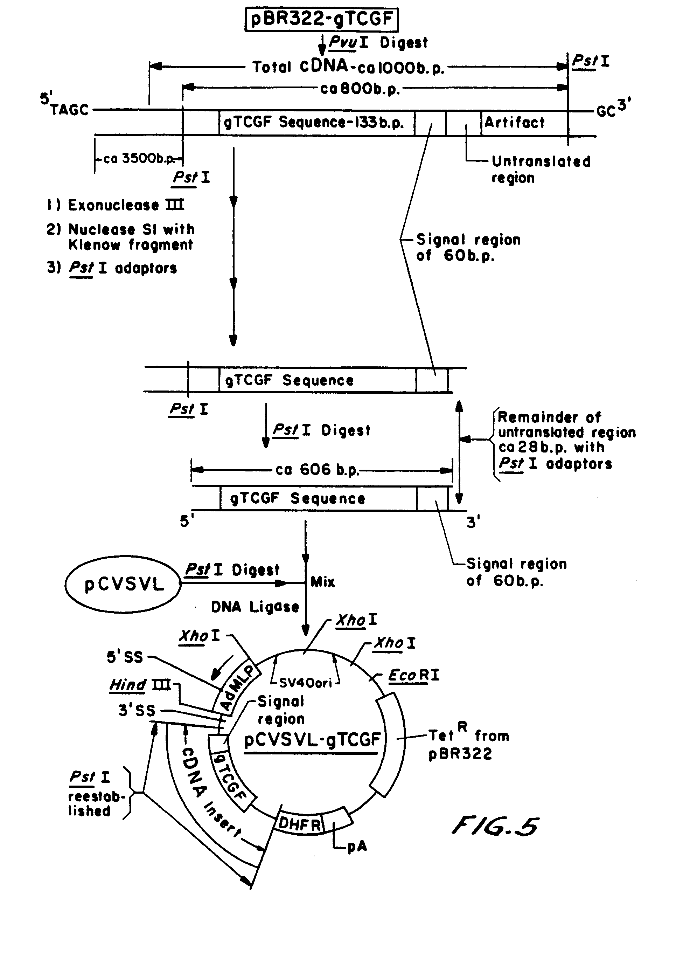

Further objects were then achieved by elucidating the nucleotide sequence of the cDNA insert in pBR322-gTCGF including all regions relevant to gTCGF, as well as the apparent sequence of protein encoded by the nucleotide sequence. As later herein detailed, the cDNA insert of pBR322-gTCGF was sequenced employing (to insure confidence) a combination of dideoxy DNA sequencing and Maxam-Gilbert specific chemical degradation DNA sequencing. The strategies represented in Fig. 2 (DNA degradation) and Fig. 3 (restriction cleavage) were both employed for obtaining cDNA fragments for dideoxy sequencing. Fig. 5 shows the restriction cleavage strategy for obtaining fragments for Maxam-Gilbert sequencing. The results of such sequencing are set forth below in Table A.

**TABLE A: Sequence of gTCGF cDNA Clone.**

```
CACTGAAGATGTTTCAGTTCTGTGGCCTTCTTGGGCATGTAAAACTTAAATGTGAGCATCCTGGTGAGTTTGGCATT

                                           ——— Artifact Region ———>|  ————
CTTGTAATTATTAATTCCATTCAAAATCATCTGTAAATCCAGCAGTAAATGCTCCAGTTGTAGCT   ATCACTCTC

— 5' Untranslated Region (47 b.p.) ——>|  |  ———— Signal Region                  —
                                      | Met Tyr Arg Met Gln Leu Leu Ser Cys Ile
TTTAATCACTACTCACAGTAACCTCAACTCCTGCCACA  ATG TAC AGG ATG CAA CTC CTG TCT TGC ATT
10    ———————————————————————————————> 20        Rsa I

Ala Leu Ser Leu Ala Leu Val Thr Asn Gly|Ala Pro Thr Ser Ser Ser Thr Lys Lys Thr
GCA CTA AGT CTT GCA CTT GTC ACA AAT GGT|GCA CCT ACT TCA AGT TCT ACA AAG AAA ACA
                                  100    40
Gln Leu Gln Leu Glu His Leu Leu Leu Asp Leu Gln Met Ile Leu Asn Gly Ile Asn Asn
CAG CTA CAA CTG GAG CAT TTA CTG CTG GAT TTA CAG ATG ATT TTG AAT GGA ATT AAT AAT
                150                 60
Tyr Lys Asn Pro Lys Leu Thr Arg Met Leu Thr Phe Lys Phe Tyr Met Pro Lys Lys Ala
TAC AAG AAT CCC AAA CTC ACC AGG ATG CTC ACA TTT AAG TTT TAC ATG CCC AAG AAG GCC
200                                 80                          250
Thr Glu Leu Lys His Leu Gln Cys Leu Glu Glu Glu Leu Lys Pro Leu Glu Glu Val Leu
ACA GAA TTG AAA CAT CTT CAG TGT CTA GAA GAA GAA CTC AAA CCT CTG GAG GAA GTG CTA
                              Xba I 100                 300
Asn Leu Ala Gln Ser Lys Asn Phe His Leu Arg Pro Arg Asp Leu Ile Ser Asn Ile Asn
AAT TTA GCT CAA AGC AAA AAC TTT CAC TTA AGA CCC AGG GAC TTA ATC AGC AAT ATC AAC
                                    120  350
Val Ile Val Gln Glu Leu Lys Gly Ser Glu Thr Thr Phe Met Cys Glu Tyr Ala Asp Glu
GTA ATA GTT CAG GAA CTA AAG GGA TCT GAA ACA ACA TTC ATG TGT GAA TAT GCT GAT GAG
                          400       140
Thr Ala Thr Ile Val Glu Phe Leu Asn Arg Trp Ile Thr Phe Cys Gln Ser Ile Ile Ser
ACA GCA ACC ATT GTA GAA TTT CTG AAC AGA TGG ATT ACC TTT TGT CAA AGC ATC ATC TCA
        153     450
Thr Leu Thr                  |———— 3' Untranslated Region ————>
ACA CTG ACT|TGA TAA |TTAAGTGCTTCCCACTTAAAACATATCAGGCCTTCTATTTATTTAAATATTTAAATTTA
500        |  Term. |                             ↑           550
                                                 Stu I
TATTTATTGTTGAATGTATGGTTTGCTACCTGTTCTGTTGTTCTGAAAGAGCTGCAGTAACGCCATTTTGCAAGGCAA
                     600                                  ↑                  650
                                                        Pst I
CGCCCAGAGCATAGGAAAAATACAGCTGAAGGAACAGGATATCTGTGGTTATGCACCTGGGCCCCGGCCAGGGCACCAT
                         ↑
                       Pvu II                  700
AGAAGACAAATAGTTCCCTAAGATAAGTCAACAGCAGTTTCCAGGGTGCCCCTCAACTGTATTAATCTGTGGTCATCGT
                  750                                                  800

TCCAGGTGTCCACACGGCCTAGGGCCAGAAGACAAATAGTTCCCCAAGATAAGTCAACACCCC
                                          850
```

Case 118-6962

In Table A, above, the first nucleotide triplet coding for Met is assigned the number 1 and the identification and numbering of amino acids ends with the codon for Thr at position 153 in view of the immediately following pair of stop signals (TGA and TAA). As might be expected, the sequence analysis indicated that the nucleotide chain of the mRNA transcribed by the gTCGF gene region is of far greater length than the nucleotide length of the coding region for the mature gTCGF protein. Hence, the analysis indicated initially that the mature gTCGF protein could have as many as 153 amino acids but from further efforts described hereinafter it is indicated that the mature gTCGF protein is composed sequentially of the amino acids numbered 21 to 153, inclusive, in Table A. The sequence also showed upstream from the first Met codon a TAA termination signal in phase with the first Met codon, thus indicating that the cDNA obtained included the whole of the gTCGF sequence. Study of the sequencing results also indicated the formation of an artifact region of some 143 nucleotides during preparation of the cDNA. This artifact region (indicated in Table A) therefore forms no part of the gTCGF gene region. Hence, the gTCGF gene region found in the cDNA begins with an upstream (5') untranslated section of 47 base pairs followed sequentially by a signal peptide sequence of 60 base pairs, the mature protein coding sequence having 459 base pairs, a pair of stop codons totalling 6 base pairs and a downstream (3') untranslated section of 309 base pairs. Also shown in Table A are various endonuclease restriction sites indicated by the revealed sequence including a Pst I site which was discovered during the sequencing work.

The invention thus provides transfer vectors containing a coding sequence for gTCGF whereby such sequence may be cloned. It also provides transfer vectors comprising a gTCGF coding sequence whereby the sequence may be expressed and production of the gTCGF protein achieved. Such transfer or expression vectors may be produced basically by placing at least the sequence coding for gTCGF in a vector such that the coding sequence is located under transcription control of a transcription control system in the transfer vector. The resulting vehicle will be a transfer vector comprising a recombinant operon comprising a coding sequence for gTCGF. By reason of its intact replicon the transfer vector will also be adapted to be suitable for replication and also for expression in a pre-selected host, e.g. animal cells or microorganisms, such as yeast or bacteria. A wide variety of vectors are available for constructing such expression vectors. In general, the particular vector to be used will depend, in addition to the matter of the host, upon various other known factors, but generally in accord with an overall strategy for expression. Commonly, many such vectors are also recombinant derivatives of other vectors, for example, sections from two or more plasmids may be combined or sections from plasmids and bacteriophages may be combined, thus providing certain desired properties while omitting sections considered less desirable for a particular purpose. The transcription control system and/or various elements thereof may be obtained from various sources which are different than the source of other sections of the transfer vector, mainly in an effort to improve expression in particular hosts. A variety of promoters, promoter-operator systems, ribosome binding site sequences and other elements are known and may be used for this purpose.

In general, the sequence in an expression vector prior to or upstream from the codon for 21-Ala, the beginning of the mature protein, may vary depending upon certain factors such as the overall strategy for finally producing gTCGF including the host to be used for expression.
Since the mature gTCGF begins with Alanine (21-Ala) rather than methionine (Met) which provides the translation initiation codon ATG in messenger RNA, it becomes in order to provide an ATG codon upstream from and in reading phase with the gTCGF coding sequence. Providing for the ATG codon at an upstream location from the 21-Ala may be effected utilizing an ATG already in a sequence comprising the gTCGF coding sequence, e.g. the gTCGF cDNA insert, or the same may be otherwise provided from an extraneous source such as the plasmid to the used for expression, provided that the ATG first appearing before the 21-Ala codon is in reading phase with the 21-Ala codon. In general, the gTCGF coding sequence will be followed by one or more codons to allow the production of a gTCGF protein which ends with last amino acid of the mature protein (153-Thr in Table A). This objective may be generally accomplished by having one or more translation termination codons immediately following the gTCGF coding sequence as represented by the sequence elucidated for the cDNA.

For purposes of producing gTCGF, the cDNA insert containing the gTCGF gene as available in the cloning vector pBR322-gTCGF may be excised therefrom and utilized in various ways. Since the sequence analysis showed the cDNA to contain a substantial nucleotide region upstream from the gTCGF coding sequence, a portion or the whole of this upstream section may be removed. Since the artifact region formed no part of the natural system, its removal is desirable in any event. Also, the whole or a portion of untranslated region of some 47 base pairs following the artifact region may be removed. The specific embodiments herein described are merely illustrative of the manner in which the cDNA insert may be used to construct transfer vectors suitable for producing gTCGF.

Expression in animal cells was the initial objective for expression in order to finally clarify the sequence of the gTCGF protein in view of the presence of the apparent signal peptide sequence (for amino acids 1-20 in Table A), since the production of the protein encoded by the sequence in animal cells afforded the opportunity that the cell system would cleave away any leading signal peptide from a precursor protein product, thus yielding the mature gTCGF. Thus, a suitable gene segment for this purpose will have at least all of the codons corresponding to amino acids 1-153 in Table A (plus the termination codons). Since it is desirable to eliminate the artifact region and since the upstream untranslated region may also be removed in whole or in part, it is apparent that the removal from the upstream end of the cDNA insert of from about 143 to 190 base pairs will provide the desired sequence for initial expression in animal cells. Since the number of nucleotides which must be removed from the cDNA insert to arrive at the desired segment is not exact, the desired segment may be prepared from the cDNA by procedures which are particularly useful for degrading more or less inexact lengths of duplex DNA.

Thus, as shown in Fig. 5, the cloning vehicle pBR322-gTCGF may be linearized by digesting with the endonuclease Pvu I. The resulting linearized DNA is then treated with Exonuclease III which degrades each strand from its 3' end. The nuclease S1 is then employed to remove the resulting extensive 5' overhangs and the Klenow fragment used to insure a blunt end. Such treatment in the exemplification hereinafter removed the entire artifact region and left 28 b.p. in the upstream untranslated section. Addition of Pst I adaptors prepares one end of the resulting degraded DNA for plasmid integration. The presence of the Pst I site within the cDNA clone affords the opportunity to liberate a segment with the desired gTCGF sequence from the remainder of the linearized cloning vehicle and create a downstream Pst I terminus about 113 b.p. from the second termination signal at the end of the gTCGF coding sequence. The resulting gTCGF cDNA segment thus contained only about 606 base pairs with a cohesive Pst I terminus at each end.

As shown in Fig. 5, the ca 606 b.p. gTCGF cDNA segment is used to form an expression transfer vector by integrating the same into the plasmid pCVSVL which has been linearized by cleavage at its sole Pst I site. The plasmid pCVSVL is known and has been described, for example, by Kaufman et al, Molecular and Cellular Biology, Nov. 1982, pages 1304-1319, the disclosure of which is incorporated herein by reference as regards the structure and properties of pCVSVL to the extent not described herein. The plasmid pCVSVL has its *Pst* I almost immediately downstream from its leftward reading (shown by arrow in Fig. 5) Adenovirus major late promoter (AdMLP) region. Immediately downstream from the *Pst* I site is the gene section for Dihydrofolate Reductase (DHFR) and the plasmid has two animal cell replicons (SV40ori) in the region of its three *Xho* I sites. As also evident from Fig. 5, the plasmid has a 5' and a 3' splice site (SS) creating one artificial intervening sequence. The processing of such a sequence from a primary transcript often substantially elevates the level of expression of a stable messenger RNA. Hence, the plasmid pCVSVL is particularly suitable for expression in animal cells upon insertion of the desired coding sequence in the correct reading orientation to the AdMLP promoter region.

The integration of the gTCGF sequence-containing segment into the *Pst* I site of pCVSVL plasmid forms the transfer vector pCVSVL-gTCGF (identified after cloning in *E. Coli*) and transfection of monkey COS-7 cells with pCVSVL-gTCGF led, after a 48-72 hour induction period, to an expression protein product which assayed positively for TCGF activity. Purification and sequence analysis of this protein product confirms the mature gTCGF product to be a polypeptide having the amino acid sequence 21-153 as set forth in Table A, and free N- and C- terminal.

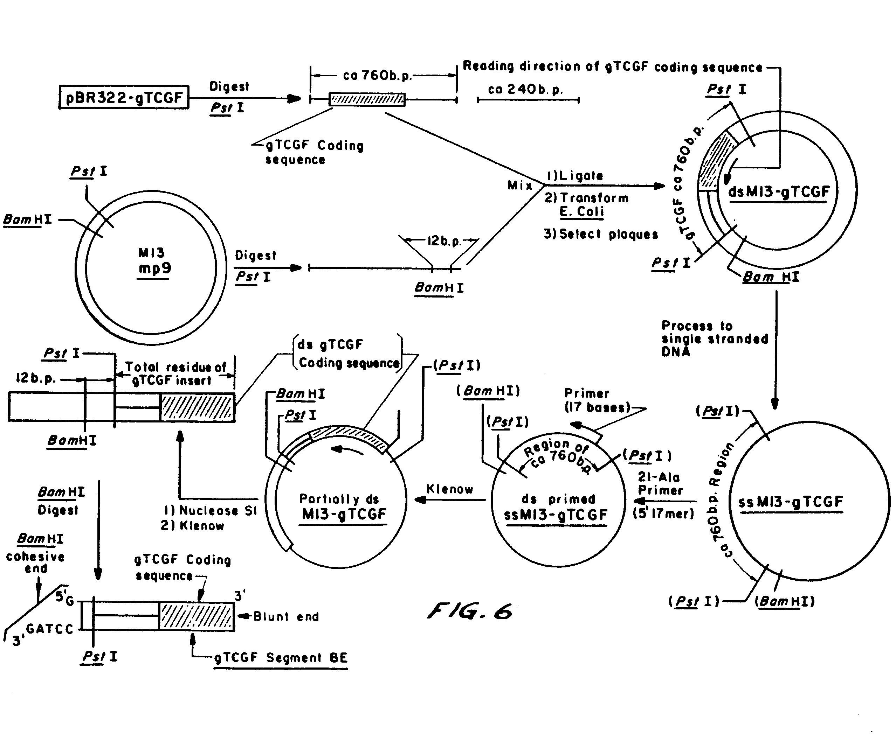

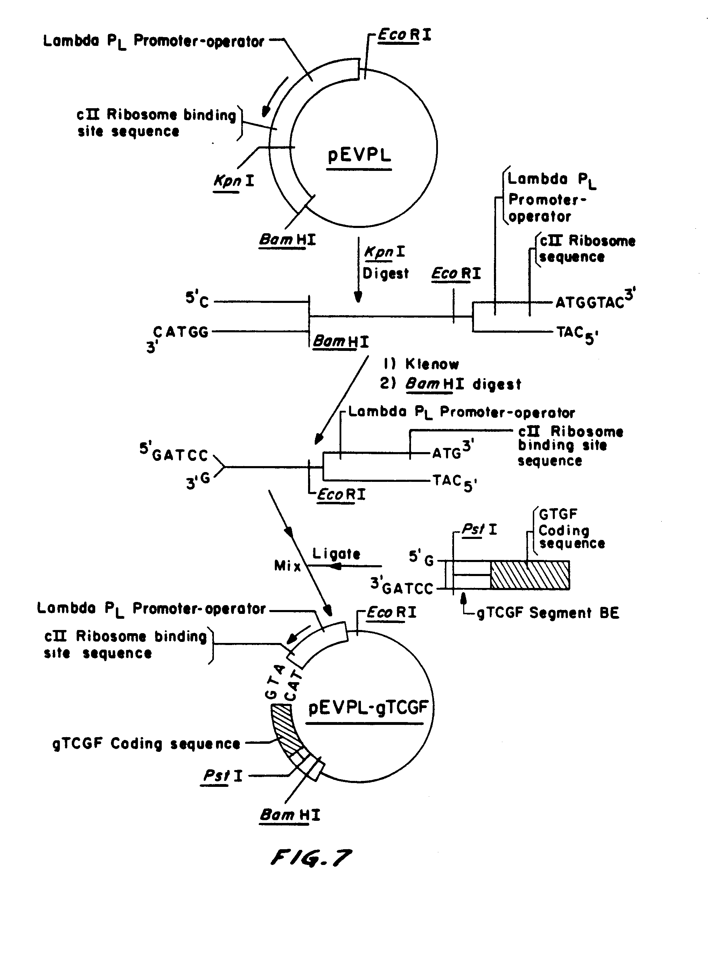

The expression of the gTCGF sequence and production of gTCGF in bacteria is represented in Figs. 6 and 7. In the embodiment shown in Figs. 6 and 7, the strategy is to employ a coding sequence in which the desired ATG initiation codon is placed upstream and immediately adjacent the 21-Ala codon, whereby upon expression in a suitable transfer vector in bacteria at least a portion of the normally encoded precursor protein (-1)-Met-gTCGF will be produced and proteolytically converted to the mature gTCGF protein product. One manner of effecting the strategy of Figs. 6 and 7 begins with the preparation of a gTCGF coding segment in which the first upstream codon is the codon for the first amino acid (21-Ala) of the mature gTCGF protein. As represented in Fig. 6, such a coding segment may be produced from the gTCGF cDNA insert available between its Pst I insertion sites in the pBR322-gTCGF cloning vehicle. Cleavage of pBR322-gTCGF with Pst I divided the insert into two segments because of the additional Pst I site within the downstream untranslated region of the cDNA. The larger segment (ca 760 b.p.) containing the gTCGF coding sequence is selected and then inserted at the Pst I site of a double stranded (ds) M13 vector, e.g. M13mp9, which has been obtained by transformation of E. Coli with the single stranded (ss) bacteriophage M13 vector and collecting the resulting ds form. Transformation of E. Coli with the resulting transfer vector dsM13-gTCGF by known procedures results in the cloning (multiplication) of the vector and the eventual discharge and recovery from the supernatant of a single-stranded cyclic DNA (ssM13-gTCGF in Fig. 6 in which the single stranded replicas of restriction cleavage sites are given in parenthesis) in which the coding strand of the gTCGF insert has been removed. Such single-stranded replica of the vector will be employed as a template for synthesis of a gTCGF coding segment which will begin at the codon for 21-Ala. For this purpose, a single stranded oligonucleotide of suitable length, e.g. a 17 mer (21-Ala primer-5' 17 mer in Fig. 6), is synthesized such that its sequence is complementary to the non-coding template beginning at the codon for 21-Ala. The balance of the oligonucleotide is constructed such that each nucleotide thereof is complementary in series to the corresponding nucleotide in the template. Hence, a suitable 17 mer will have the structure 5' d(GCACCTACTTCAAGTTC). Mixing of the oligonucleotide with the single-stranded template (ssM13-gTCGF) results in the oligonucleotide hydridizing to the first 17 nucleotides of the template corresponding to the gTCGF coding sequence and beginning with the first template nucleotide complementary to the first nucleotide of the 21-Ala codon. Such hydrization effectively provides a primer site for synthesis of additional portions complementary to the template by treating with the Klenow fragment in the known manner. Such synthesis of the complementary strand is sufficiently effected or continued well beyond the BamHI site provided by the M13 vector. Treatment of the resulting product with the Nuclease S1 removes the remaining single stranded portions and the resulting DNA which is now linear is treated with the Klenow fragment with all four deoxynucleotides added to insure the formation of a gTCGF code-containing duplex which begins exactly with a blunt end coding for 21-Ala. The final gTCGF gene duplex (gTCGF Gene Segment BE in Figs. 6 and 7) is obtained by cleaving the linear DNA with BamHI which cleaves in the so-called M13 polylinker section adjacent to the reestablished Pst I where the gTCGF cDNA was inserted into the dsM13 vector. Hence, the final gTCGF coding duplex has about 530 base pairs and a 5' blunt end beginning with the codon for Ala and at the other end a cohesive terminus from the BamHI cleavage.

In the strategy represented in Figs. 6 and 7 the objective of having the initiation triplet ATG coding for Met at the beginning of the mature gTCGF coding sequence is accomplished by making the ATG available from the plasmid to be used to form the recombinant expression vector for transforming the bacterial host. As shown in Fig. 7, the desired ATG codon may be provided in the ultimate expression transfer vector for producing gTCGF by employing a plasmid such as pEVPL. This plasmid has a lambda $P_L$ promoter-operator upstream from the cII ribosome binding site sequence that is immediately followed by a nucleotide section terminating in a Kpn I site. This section begins with the ATG translation initiation codon of the cII coding sequence (which otherwise has been removed) and the Kpn I site partially overlaps this desired ATG sequence (underlined below), said section with its actual Kpn I cleavage locations shown by arrows being:

```
            ↓
5'  ——ATGGTACC——  3'
3'  ——TACCATGG——  5'
       (↑)
      Kpn I
```

Cleavage of pEVPL with Kpn I creates a 3' GTAC overhang which can be readily trimmed back to the blunt ended G-C base pair terminal of the desired ATG codon by employing the Klenow fragment of DNA polymerase. The plasmid pEVPL also contains a conveniently located BamHI site which may then be cleaved to enable the integration of final gTCGF Segment BE into the linearized pEVPL by ligating (T4 ligase) the blunt end ATG of the plasmid to the blunt end GCA (21-Ala) of the gene segment BE and ligation (T4 ligase) of the complementary cohesive BamHI termini of the respective parts. The resulting transfer vector (pEVPL-gTCGF with its reading direction shown by arrow in Fig. 7) is then used to transform in a conventional manner a suitable bacterial host such as E. Coli which has been modified to carry a lambda lysogen which has a temperature sensitive repressor sequence and which is defective in bacterial lysis, such as E. Coli W3110 lambda Y139. The resulting transformant may be isolated in the conventional manner and a portion tested for expression using cells labelled with $^{35}$S-methionine as described hereinafter in Example B. Having obtained a positive indication of expression of desired protein (Example B), the balance of the transformed cells may be subjected to expression by growing the cells for several generations (about 2 hours) at 30°C., then raising the temperature to 42°C. for 10 minutes and then lowering the temperature to 37°C. for 2 hours. Assay of sonicated extracts of the cells reveals TCGF activity but it has been found that most of the gTCGF produced is insoluble in the cytoplasm. However, the insoluble active protein can be removed and further concentrated by centrifugation, washing the pellet with aqueous (10 mM Tris HCl, pH 7.5, and 1 mM EDTA) buffer and dissolving in SDS (0.1%). About 50-70%, e.g. 60% of the protein product obtained in this manner is product from expression of the sequence containing the gTCGF sequence. This product may be sequenced using a gas-phase micro-sequentor after fractionation by preparative SDS gel electrophoresis and cutting out and eluting the band corresponding to a protein standard of known and similar molecular weight. In this manner it may be confirmed that a high proportion, e.g. 85-98%, of protein eluted from the gel was product from the expression of the sequence containing the gTCGF sequence. At least a significant portion of such expression product, typically 20-50%, conforms to the 133 amino acid structure of gTCGF beginning with Ala-Pro-Thr- as shown in Table A. The balance, typically 50-80%, represented the incompletely processed precursor of 134 amino acids beginning with Met-Ala-Pro-Thr. Expression of the gTCGF coding sequence in bacteria under the influence of the Lambda $P_L$ promoter-operator, especially when employing other control elements provided by the transcription control system of the plasmid pEVPL, including particularly the cII gene ribosome binding site sequence, results in the production of the desired expression product in high quantities. For example, in a small scale (10 liter) fermentor with the strain W3110 lambda Y139 it is indicated that expression product yields may be obtained that are of the order of at least 100 mg./liter of total fermentation mixture, more typically about 200 mg./liter, at a relatively low cell density of 10 dry grams per liter. Since cell densities can be increased such as to the order of 20-30 dry grams per liter, production of the desired expression product may be increased to even higher levels of the order of at least 400-600 mg./liter.

The expression of pEVPL in E. Coli W3110 Lambda Y139 is therefore representative of an embodiment of the invention in which the coding sequence for the repressor protein for the expression vector operator is incorporated within the chromosome of the host bacteria. Procedures for modifying bacteria in this manner are known. For example, the Lambda Y139 lysogen (available NIH Lambda Phage Collection) may be used to prepare phage stock in E. Coli C600 by the method described by Maniatis et al., Molecular Cloning, A Laboratory Manual, Cold Spring Harbor Laboratory (1982). The E. Coli W3110 is grown in L-broth (60% Bacto-tryptone, 0.5% Bacto-yeast extract, 0.5% NaCl) containing 10 mM $MgSO_4$ and 0.5% maltose to a cell density of $3 \times 10^8$ cells/ml and mixed with phage stock at a multiplicity of infection of 5. The infected cells are allowed to stand at room temperature for 30 minutes, serially diluted into tubes of L-broth and samples from each tube plated on T-plates and incubated at 30°C. until colonies are found.

The desired colonies are those able to support growth of wide type Lambda at 42°C. but not at 30°C.

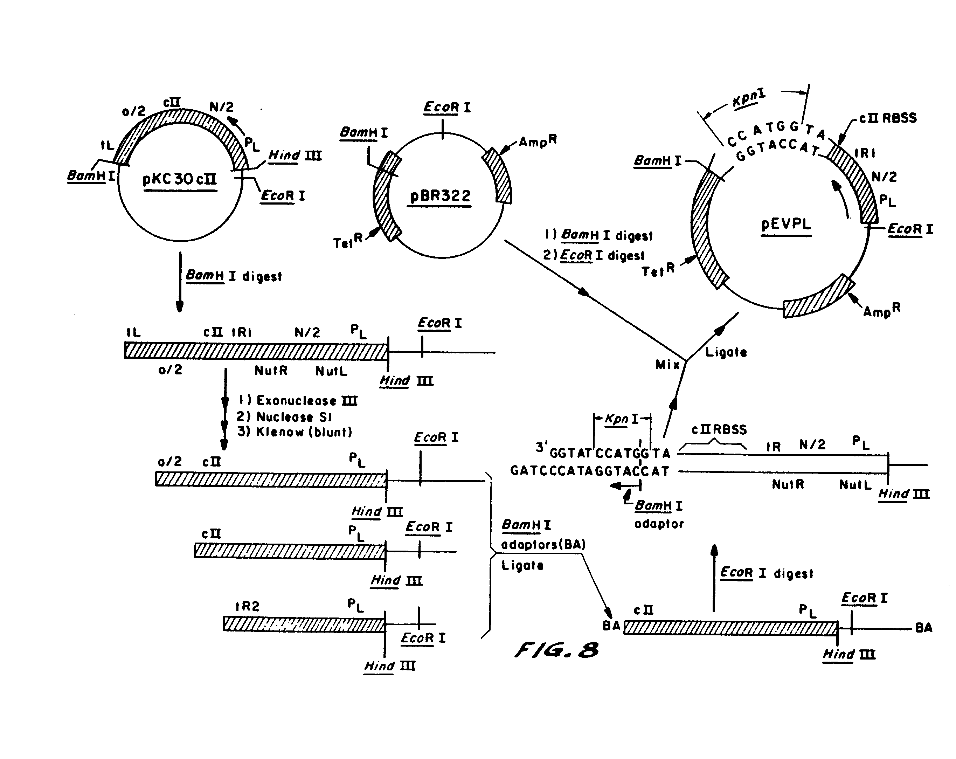

The plasmid pEVPL is novel and its preparation from a known plasmid is described in detail hereinafter in Example A with reference to Fig. 8.

The gTCGF produced by the invention may be used in a variety of applications as known and indicated in the literature for this lymphokine and other mammalian T-cell growth factors which have the capacity to support the replication of activated T-cells of mammalian species. These applications include use to promote growth of animal cells in culture and other *in vitro* applications and also include therapeutic use in treating a variety of conditions. Therapeutic applications of particular interest are based on the recognized immunoregulatory properties of the lymphokine, in particular its use in treatment of conditions due to immunodeficiencies such as the severe combined immunodeficiency syndrome (SCIDS), the acquired immunodeficiency syndrome (AIDS), immunodeficiency states of old age and congenital immunodeficiency. However, the gTCGF may also be used as an anti-micotic agent in the treatment of various viral diseases such as cytomegalovirus infections and Herpes virusinfections and in the treatment of bacterial infections such as Tuberculosis and Leprosis. Dosages required for such therapeutic use of gTCGF will vary depending upon known factors such as the condition being treated, the severity of the condition and the like. However, satisfactory results may be generally obtained when administered to mammals, e.g. human patients, in daily dosages ranging from 0.1 μg to 30 μg/kilogram of body weight. Intraveneous administration in a suitable sterile vehicle is generally preferred.

Among the wide variety of particular uses of gTCGF based on its ability to promote T-cell growth in culture are those which find application in diagnostics and in assisting therapeutic treatments. Use in diagnostics is based on the known ability to detect the presence of certain diseases by determining the extent of T-lymphocite growth in vitro after addition of the lymphokine, using the uptake of radioactive thymidine in the cells as a measure. In assisting therapeutic treatments, such as anti-tumor treatment, the gTCGF may be used to promote in vitro the growth of T-lymphocites obtained from the patient or other compatible donor, and the resulting proliferated lymphocites then reinfused to the patient to assist in combatting the condition. In general, the amount of gTCGF to be used to support T-cell growth in culture and other in vitro applications may be generally determined from literature accounts of its activity and of its comparison to human TCGF in such systems, see for example, Rabin et al., supra, and may be optimized for particular situations by routine investigations. Mixtures of the (-1)-Met-gTCGF and the mature gTCGF protein may be used in the same manner and at dosages or concentrations similar to those of the mature gTCGF protein based on the indication that the (-1)-Met-gTCGF protein exhibits about the same level of activity as the mature gTCGF protein.

It will be appreciated that the gTCGF which may be produced in accordance with the present invention and the DNA sequences coding therefor may correspond to any of the structural variations discussed previously, in particular the amino acids 1 to 153 of Table A, the amino acids 2 to 153 of Table A, the amino acids 21 to 153 of Table A and the amino acids 21 to 153 of Table A immediately preceded by Met (i.e. Met-gTCGF). Moreover, naturally-occurring allelic variations of gTCGF may likewise be isolated using the techniques described and in particular using hybridisation probes based on characteristic nucleotide sequences in the structures disclosed. Furthermore, the various DNA sequences disclosed can be modified by conventional techniques, to produce variations in the final gTCGF protein, for example amino acid sequences in which one or more, in particular 1, 2, 3, 4 or 5, amino have been removed, added or replaced and yet the final gTCGF protein still exhibits gTCGF activity. Belgian Patent 898,016, which is incorporated herein by reference describes one such typical technique for replacing cysteine by e.g. serine. This may remove the possibility of undesired or unnecessary cysteine disulphide bridging and may result in products of greater stability.

It will be appreciated that all such modifications are within the scope of the present invention as well as the DNA sequences coding for them and transfer vectors comprising the latter.

The following examples are representative and designed to illustrate in detail the manner in which certain embodiments of the invention may be practiced. As will be appreciated, work of the type described in the examples is conducted on a very small scale, often involves mixtures, typically relies on the law of probability and fine detection analysis to ascertain successful results and in some cases cannot be exactly duplicated in every fine detail due to, in particular, the natural source or living nature of materials used or produced. Accordingly, it will also be appreciated that reasonably limited repetitions of correct procedures may be occasionally required in reproducing the desired objectives of successful experiments in this field. Temperatures are in °C. unless otherwise noted. In the examples, unless otherwise specified, the TCGF assay used is a thymidine incorporation assay using a mouse cell line (CTLL-2) which is completely dependent on TCGF for growth. In this assay 10'000 CTLL-2 cells are seeded in 100 ul RPMI 1640 with 2% fetal calf serum in 96-well flat-bottom microplates together with 100 ul of the serially diluted samples to be assayed. After 20 hours at 37°C, cells are pulsed for 4 hours with 0.5 uC of $^{3}$H-thymidine, collected on glass fiber filter strips using an automatic cell harvester and the incorporated radioactivity determined by liquid scintillation counting. In this assay, the dilution of a laboratory standard that stimulates the incorporation of 50% of the maximal (saturating) levels of 3H-thymidine is arbitrarily assigned a value of 1 unit/mL. The activity of each unknown is compared to the laboratory standard.

EXAMPLE 1

Step A: mRNA Preparation From The Gibbon T-cell Line

A sample of the Gibbon T-cell line designated UCD-MLA 144 (about 2 x $10^6$ cells) was cultured for several weeks in RPMI 1640 (purchased from Gibco) + 20% FCS (fetal calf serum) until there was obtained 1 x $10^9$ total cells. The cells were induced to produce high levels of gTCGF by activation for 24 hours in the presence of 10 nanograms per ml 12-0-tetradecanoyl phorbal 13-acetate (TPA) in RPMI 1640 + 1% FCS. The cells were harvested by centrifugation (1000 rpm 5 min.), washed once with PBS and finally collected by centrifugation. Cytoplasmic RNA was prepared by a gentle lysis procedure in which the cells were resuspended in 50 ml cold Triton lysis buffer (140 mM NaCl, 1.5 mM $M_5Cl_2$, 10 mM Tris, pH 8.6, 0.5% Triton X-100) with 10 mM dithiothirectol (DTT) and 50 units/ml RNAsin (purchased from Biotech). This lysate was divided into 2 equal parts and each part was layered over a 10 ml cushion of lysis buffer containing 20% sucrose. The cell nuclei were removed by centrifugation in the cold (4$^o$C.)-(4000 rpm for 5 minutes). The upper layer (cytoplasmic extract was carefully removed and sodium dodecyl sulphate (SDS) was added to a final concentration of 1%. This solution was extracted 2 times with an equal volume of phenol chloroform (1:1 mixture) and the RNA was precipitated by adding 2.5 volumes of cold ethanol. The precipitated RNA was collected by centrifugation (15 min. at 4000 rpm) and resuspended in 0.01 M Tris, pH 7.5, 1 mM EDTA, 0.25 M NaCl (TE buffer plus .25 M NaCl) and reprecipitated by addition of 2.5 vol of cold ethanol. Finally, the RNA was collected by centrifugation and resuspended in 5 ml of $H_2O$. The final yield was 17.5 mg. Messenger RNA was isolated from the total cytoplasmic RNA by selection on oligo dT cellulose. 2.5 mg of total RNA was heated to

65° for five minutes. NaCl was added to 0.5 M and the RNA was allowed to cool to room temperature. This RNA was passed over a one ml column of oligo dT cellulose equilibrated in TE + 0.5M NaCl (Binding buffer). Unbound RNA was removed by washing the column extensively with binding buffer. Bound messenger RNA was eluted with 3 ml of $H_2O$ and precipitated by addition of 0.2 ml of 4 M NaCl and 2.5 volumes of cold ethanol. The precipitated mRNA was collected by centrifugation (30 minutes at 25,000 rpm). The final pellet (approximately 100 ug) was resuspended in 50 ul $H_2O$. Confirmation that the final mRNA preparation contained biologically active gTCGF mRNA was accomplished using the standard Xenopus laevis oocyte translation system in which 10 fresh oocytes isolated from female African toads (Xenopus laevis) were each microinjected with 40 nanoliters of the UCD-MLA 144 mRNA (1 nanogram per nanoliter). Control oocytes were microinjected with $H_2O$. The oocytes were incubated for 24 hours at 20°C in 100 ul Barth's medium. (Maniatis et al. supra p. 351)

During this period, each oocyte injected with gibbon mRNA should translate, produce and finally secrete TCGF into the medium while the water injected controls should not. TCGF activity was determined using a standard assay protocol, and it was found that the Xenopus oocyte injected with gibbon mRNA synthesized significant levels of TCGF activity while the water-injected controls did not.

Step B: First strand cDNA Reaction

20 ug of UCD MLA-144 mRNA in 10 ul of $H_2O$ was treated with 0.1 M methyl mercury for 10 min. at room temperature. The methyl mercury was inactivated by addition of 10 ul of 100 mM β-mercaptoethanol. This denatured mRNA was diluted into a 100 ul cDNA synthesis reaction containing 100mM Tris pH 8.4, 140 mM KCl,

10 mM $MgCl_2$, 10 mM β-mercaptoethanol, 500 uM each of dATP, dGTP, dCTP and TTP, 5 μg. of oligo-dT (average size 12-18) as primer, 75 uCi of $^{32}P$dCTP (400 Ci/mmole)and 20 units of the ribonuclease inhibitor RNAsin. The reaction was initiated by addition of 40 units of reverse transcriptase at 37°C and incubated for 30 minutes at 42°C. The reaction was stopped by addition of EDTA to 40 mM and extraction with an equal volume of $H_2O$ saturated phenol. The phenol phase was back extracted with 50 ul of TE buffer. The aqueous phases were pooled. The cDNA/RNA hybrids were separated from unincorporated triphosphates by passing the pooled aqueous phase over a 5 ml sepharose CL-4B (purchased from Pharmacia) column equilibrated with TE. The fractions that were excluded from the column were pooled, brought to 250 mM NaCl and the nucleic acids precipitated by addition of 2.5 volumes of cold ethanol. The hybrids were collected by centrifugation for 30 minutes at 25,000 rpm. The final pellet (2.5 ug of cDNA) was resuspended in 50 ul of $H_2O$.

Step C: Second strand cDNA reaction

Second strand cDNA was synthesized by the combined action of the enzymes E.coli DNA Polymerase I, E.coli DNA ligase and E.coli RNAse H. The reaction mixture (50 ul) contained 20 mM Tris, pH 8.0, 4 mM $MgCl_2$ 1.2 mM EDTA, 25 uM NAD, 100 uM each of dATP, dGTP, dCTP and TTP and 50 uC $^{32}P$ dCTP (400 Ci/mmole). The reaction was performed by adding 3 units DNA polymerase I, 0.5 units DNA ligase, and 0.75 units of RNAse H and incubating at 16° for 1 hour and then for a second hour at 37°. The reaction was stopped by adding EDTA to 40 mM and extracting with an equal volume of phenol. The phenol phase was back extracted with 50 ul TE, the aqueous phases pooled and the cDNA was separated from the unincorporated triphosphates by chromatography on a sepharose CL-4B column as described above for the first strand. Based on incorporation of $^{32}P$, the first strand cDNA was quantitatively converted to a double-strand loopback form.

Step D: S1-nuclease digestion

The loopback cDNA was converted to a clonable form by cleaving the loop with nuclease S1. To do this 0.75 ug of double strand cDNA in a 300 ul reaction containing sodium acetate pH 4.5, 1 mM Zn Acetate and 0.1 M NaCl was incubated for 30 minutes at 30° with 12 units of S1. The reaction was terminated by extraction with an equal volume of phenol. The phenol phase was back extracted with 50 ul of TE and the pooled aqueous phases were passed over a 5 ml sepharose Cl-4B column as described above. Fractions of 150 ul were collected and the first three, (the excluded cDNA fractions) were pooled and the nucleic acids precipitated by addition of NaCl to 0.25 M and 2.5 volumes of cold ethanol. The cDNA was collected by centrifugation (30 min. at 25,000 rpm) and resuspended in 45 ul of $H_2O$. The final yield was 340 ng of cDNA.

Step E: Recombinant cDNA Preparation

Homopolymeric C "tails" were added to the ends of the cDNA by gently heating 100 ng of cDNA in a 50 ul reaction mixture containing 0.1 M β-mercaptoethanol 1 mM $CoCl_2$ and 6 units of terminal deoxynucleotidyl transferase at 37°C for 5 minutes. The reaction was terminated by the addition of EDTA to 40 mM and heating to 68°C for 10 minutes. 10 ng of this tailed cDNA was annealed with 50 ng of G-tailed pBR322 which had been linearized by digestion with the restriction endonuclease Pst 1 (and tailed with dGTP as described above for the C-tailing of the cDNA) in 50 ul of 10 mM Tris, pH 7.5, 1 mM EDTA, and 100 mM NaCl. The annealing reaction was performed at 50°C for 2 hours after a 5 minute preincubation at 68°C. Steps B-E, above, are represented in Fig. 1.

Step F: Bacterial transformation

The cDNA annealing reaction product was used directly to transform the E.coli strain MC1061. A fresh colony of bacterial cells were used to inoculate 50 ml of L-broth and grown for several hours until the optical density at 550 nm was 0.25. The cells were chilled on ice and harvested by centrifugation (4000 rpm for 10 min.). The pellet was resuspended in 10 ml of cold 0.1 M $CaCl_2$ and allowed to sit on ice for 10 min. The cells were collected by centrifugation (4000 rpm for 5 minutes) and resuspended in 2.5 ml of 0.1 M $CaCl_2$. 10 ul of the cDNA annealing reaction was then incubated with 200 ul of $CaCl_2$-treated bacteria for 30 minutes on ice and then for 2 minutes at 37°C., followed by addition of 0.8 ml of L-broth and final incubation for 30 minutes at 37°C. Five of these transformations were performed utilizing all of the annealed cDNA. Each transformation mixture was spread directly onto nitrocellulose filters placed on the surface of standard 1% Agar L-broth plates (10cm diameter) containing 10 ug per ml tetracycline. From the five transformations a total of 30 such plates were spread and incubated overnight at 37°C. On the average approximately 250 bacterial colonies grew on each plate for a total of 7,500 clones.

Step G: Replica Plating

Two identical replicas were prepared from each filter (Step F, above) by standard replica plating methods, in which each filter from the original library (the master filter) was carefully removed from its agar dish and placed colony side up on a sterile square of filter paper (Whatman 3 MM) resting on a square piece of glass. A new, pre-wetted nitrocellulose filter was carefully aligned on top of the master filter, covered with a second sterile square of filter paper and the complete sandwich then pressed together firmly with a second piece of glass. The sandwiched filters were numbered and 3 pinholes were punched through them asymmetrically so that they could be exactly aligned again in the future. The replica was then removed from the master and placed colony side up on a new tetracycline-containing L-broth agar plate. A second replica was immediately prepared in identical fashion. Each master filter was returned to its respective petri dish and all of the plates were incubated at 37° for several hours until the bacterial colonies had reached approximately 1 mm in diameter. At this time, all of the replica filters were transferred to fresh agar plates containing L-broth plus 10 ug per ml tetracycline and 150 ug per ml chloramphenicol. These plates were incubated overnight at 37°C to allow the plasmid in each colony to amplify. The original masterplates were stored at 4°C.

Step H: Preparation of filters for hybridization

Each replica filter (Step G) was placed (colony side up) on filter papers (Whatman 3mm) soaked in 0.5 M NaOH, 1.5 M NaCl for seven minutes. The filters were transferred to neutralization filter papers (soaked in 1 M Tris, pH 7.5, 1.5 M NaCl) for 2 minutes and then transferred to a second set of neutralization filters for 5-10 minutes. Finally, the filters were placed on filters soaked in SSC buffer (0.015 M Sodium Citrate, 0.15 M NaCl, pH 7.4) for 5 minutes, air-dried and baked in vacuo at 80°C for 1-2 hours.

Step I: Hybridization Probe Preparation

Two independent 17 nucleotide probes were chemically synthesized employing the known solid phase phosphite Triester method. The first probe intended as a 5' probe conformed to the structure d(G-C-A-C-C-T-A-C-T-T-C-A-A-G-T-T-C) and the second probe (a 3' probe) conformed to the structure d(C-T-G-A-T-T-A-A-G-T-C-C-C-T-G-G-G). Each such 17 oligonucleotide probe was labeled at its 5' end using $\gamma^{32}$P-ATP and polynucleotide kinase by forming a reaction mixture (25 ul) contained 10 pM of oligonucleotide, 10 pM $^{32}$P-ATP (300 Ci/mmole) 50mM Tris, pH 7.6, 10 mM $MgCl_2$, 5 mM DTT and 10 units of T4 polynucleotide kinase. The reaction mixture was incubated at 37°C for 30 minutes and stopped by the addition of TE + 0.1 M NaCl to 100 ul and an equal volume of phenol: $CHCl_3$ (1-1 mix). The aqueous layer was passed over a 5 ml Sephacryl S-200 (purchased from Pharmacia) column equilibrated in TE to remove unincorporated label.

Step J: Screening the cDNA library filters

Each set of duplicate filters was incubated at 68°C for 1 hour in pre-hybridization mix [4 x SSC, 100 ug/ml Herring sperm DNA, 5X Denhardt's solution, (0.2% Ficoll, purchased from Sigma, 0.2% polyvinyl-pyrrolidone, 0.2% Bovine Serum Albumin) and 0.1% SDS)]. After the prehybridization, bacterial debris were removed from each filter by wiping with a tissue. One set of filters was annealed with the 5' specific $^{32}P$-labeled probe while the other set was annealed with the 3' specific probe. The annealing reactions were performed in the pre-hybridization mix (as above) with the addition of 100,000 cpm per ml of labeled probe. Each set of filters was incubated in these reactions overnight at 30°C with gentle shaking. The filters were then washed with several changes of 4XSSC +0.1% SDS at room temperature for 1 hour and then exposed to x-ray film for 6-12 hours at -70°C with intensifying screens. By lining up the x-ray film exposures of the duplicate filters, it was seen that a number of colonies hybridized to one or the other hybridization probe but only one colony hybridized with both (out of approximately 7,500 colonies). The film of this filter was aligned with the original master filter containing the live bacterial colonies from this alignment. The clone which hydridized with both probes was found in a cluster of three colonies and all three such colonies were picked for further analysis.

Step K: Analysis of the clones

Each of the three colonies (Step J) was inoculated into tubes containing 2 ml of L-broth +20 ug/ml tetracycline and grown overnight at 37°C. Plasmid DNA was prepared from these cultures using a standard rapid DNA preparation method. To do this, the bacterial cells were harvested by centrifugation (2 minutes in the microfuge) and resuspended in 100 ul of 50 mM glucose, 25 mM Tris, pH 7.5, 1 mM EDTA containing 0.25 mg lysozyme at 0° for 30 minutes. Next, the bacteria were disrupted by the addition of 200 ul 0.2 M NaOH 1% SDS at 0° for 5 minutes. The lysate was neutralized by addition of 150 ul of 3 M sodium acetate, pH 4.8 and allowed to sit on ice for 15 minutes. The precipitate of denatured protein and chromosomal DNA was removed by centrifugation (10 minutes in the microfuge at 4°C). The supernatant was extracted once with phenolchloroform (1:1 mix) and the nucleic acids precipitated by the addition of 2.5 volumes of cold ethanol. The nucleic acids were collected by centrifugation (10 minutes in the microfuge) resuspended in 250 ul of 0.1 M sodium acetate, 50 mM Tris pH 8 and reprecipitated by the addition of 2.5 volumes of cold ethanol. The final plasmid DNA was resuspended in 50 ul of TE and a portion used to form a reaction mixture (15 ul) containing 0.05 M Tris, pH 8, 10 mM $MgCl_2$ 50 mM NaCl, 7 ul of the plasmid DNA and 2 units of the restriction endonuclease Pst 1. After 1 hour at 37°C bromphenol blue (tracking dye) was added to 0.005% and each reaction mix was electrophoresed on a small TBE (50 mM Tris-Borate, pH 8.3, 1 mM EDTA) agarose gel containing 1 ug/ml Ethidium bromide for 2 hours at 100 volts. The DNA fragments were visualized by illuminating the gel with ultraviolet light and the results recorded by taking a polaroid photograph of the irradiated gel. Because the cDNA clones were constructed by inserting C-tailed cDNAs into a G-tailed pBR322 vector (linearized at the Pst 1 site), digestion of each plasmid with Pst 1 should excise the insert from the plasmid vector allowing a determination of the cDNA insert size. Agarose gel electrophoresis of the three Pst 1 cut plasmids revealed that clone designated clone 2 had an 850 base pair insert fragment while the inserts of clones 1 and 3 were either much smaller (clone 1) or much larger (clone 3). To confirm that clone 2 was the clone originally identified by colony hybridization, the DNA from the Pst 1 digestions were analyzed by Southern Blot hybridization procedure to the original 17-mer oligonucleotide probes. In such procedure the agarose gel was treated with 0.5 M NaOH, 1.5 M NaCl for 30 minutes at room temperature to denature the DNA in the gel. The gel was neutralized by gently shaking it in excess 1 M Tris, pH 7.5, 1.5 M NaCl. Two nitrocellulose filters were cut to the size of the gel, wetted with 2 x SSC, and carefully placed on both sides of the gel. The gel, sandwiched between the 2 filters, was placed on top of a stack of paper towels (about 2 cm high) and covered with another layer of paper towels (also about 2 cm). Finally, the whole assembly was covered with a small glass square which was weighted in place with about 500 grams and allowed to stand overnight. By this method, liquid leached out of the gel in both directions into the paper towels. DNA present in the gel also transfers out of the gel but is retained on the nitrocellulose filters. The filters were then used in hybridization experiments in which the filters are air dried, baked in vacuo (80°C for 1 hour), incubated in pre-hybridization mix (Step J, above) at 68°C, for 1 hour and each filter then hybridized with one or the other of the original chemically synthesized probes (prehybridization mix plus $10^6$ cpm/ml $^{32}$P-labeled oligonucleotide; 30°C for 2 hours). After hybridization, the filters were rinsed for 30 minutes with 4 x SSC + 0.1 % SDS and exposed to X-ray film. The 850 base pair insert from clone 2 hybridized to both probes while the inserts from clones 1 and 3 did not.

**STEP L: Sequencing of the cDNA of the candidate clone was then carried out in accord with two procedures involving a) dideoxy DNA sequencing; and 2) Maxam Gilbert sequencing, as follows:**

a) by dideoxy DNA sequencing, the general details and procedures thereof being described in the M13 Cloning Dideoxy Sequencing Manual published by Bethesda Research Laboratories, Inc., Bethesda, Md (1980). Inserts for subcloning into M13 vectors were prepared by both of the methods: a-1) nucleotide deletion (degradation); and a-2) restriction endonuclease cleavage.

In method a-1) as shown in Fig. 2, the cloned DNA (pBR322-gTCGF) was cleaved with Pvu I (i.e. 126 nucleotides from the Pst I site where the cDNA insert was integrated into pBR322) and 5ug of the Pvu I linearized DNA was incubated at 30° with 600 units of Exonuclease III (to degrade each strand of the duplex DNA sequentially from its 3' ends) in 100 ul of 50mM Tris, pH 8 10mM 2-mercaptoethanol and 5mM $MgCl_2$. Then 14ul samples of the reaction were removed after 1 min, 2 min, 3 min, 4 min and 5 min and added to a tube containing 300 ul of ExoIII quench (20mM EDTA, 0.5M NaCl). The degraded and ethanol precipitated DNA was then incubated in 160ul of 30mM Na Acetate, pH 4.6, 200mM NaCl, 1mM $ZnSO_4$ and 40 units of S1 nuclease. The reaction was stopped after about 30 minutes at 30° by the addition of 40 ul of 0.5M Tris-HCl, pH 8.0, 1M NaCl. The ethanol precipitated and collected DNA was then incubated in 100 ul of 10mM Tris-HCl pH 7.5, 100uM each of dATP, dCTP, dGTP and dTTP, 1mM dithiothreitol, 10mM $MgCl_2$ and 5 units of Klenow fragment of DNA polymerase I for 15 min. at 37°. The reaction was stopped by addition of NaCl(4M) to final 0.2M concentration and extraction with phenol/chloroform. The resulting blunt ended DNA segments of varying length (represented by the five segments in Fig. 2) were collected by ethanol precipitation and ligated to BamHI adaptors prepared by treating 300pmole of the 11-mer with T4 polynucleotide kinase and 1mM ATP, stopping the reaction by extraction with phenol/chloroform and chloroform, adding 300pmole of the 15-mer, and ethanol precipitating.

The 15-mer plus 11-mer were resuspended in 30ul of 10mM Tris, pH 7.5, 1mM EDTA, 0.2M NaCl and annealed at 15° for 2 hours. The adaptor structure is as follows:

5' HO-G-A-T-C-C-G-C-G-G-C-G-G-T-A-C 3'

3' G-C-G-C-C-G-C-C-A-T-G$_p$ 5'

The adaptor (15pmole) was then incubated with the degraded and blunted DNA in 30ul of 15 mM Tris.HCl pH 7.5, 10mM $MgCl_2$, 10mM DTT, 100uM ATP and 0.1 unit of T4 ligase at 15° for 90 minutes. The reaction was stopped by heating at 68° for 15 min. The "Bam-adapted" DNA was then digested with the Pst I (20 units) for 4 hours at 37° after adding NaCl to 66mM (final volume 31ul). Pst I will cleave the DNA at any of the three Pst 1 recognition sites not deleted from the clone. Any fragments in which the Pst I site at the 5' end of the clone has been deleted will have a BamHI adaptor ligated to the end of the deletion and will extend to the Pst I site in the 3' non-coding region of the clone (Fragments shown in Fig. 2 ). To isolate fragments of the appropriate size, the Pst I digestion reaction was fractionated by electrophoresis through a 1% tris-acetate (0.04M Trisacetate, pH 3, 0.00 mM EDTA) agarose gel in the presence of ethidium bromide. Five size classes of the deleted DNA were isolated by carefully cutting and isolating five separate regions of the gel between 200 and 800 nucleotides using the glass powder isolation protocol, see below. As indicated in Fig. 2, the bacteriophage M13 vector mp9 (100 ug) was prepared by digesting with 150 units of BamHI and 150 units Pst I. The linearized mp9 vector DNA was separated from the resulting small oligonucleotide fragment by passing the reaction mix (100ul) over a 2ml gel filtration column (Sepharose CL-4B) equilibrated in 10mM tris.HCl pH 7.5, and 1mM EDTA (TE). The excluded fractions were pooled, brought to 0.4M NaCl, precipitated with ethanol, resuspended in 100ul of TE and the linearized M13mp9 vector preparation diluted 25 fold (to 40ng/ul). As indicated in Fig. 2, each (5ul) of the 5 pools of degraded cDNA fragments was ligated to the linearized M13mp9 vector (20 ng) in a 20ul ligation mix. After 90 min at 15° the reaction was terminated by heating at 68° for 15 minutes. E. coli strain JM101 (45 ml) which had been exponentially growing in SOBM medium (20g tryptone, 5g yeast extract, 0.58g NaCl and 0.11g KOH, 10mmole $MgSO_4$ per liter) were collected by centrifugation (10 min. at 2500 rpm at 4°C). The pellet was resuspended in 3.1 ml of cold Tfb (100mM RbCl, 45mM $MgCl_2$, 50mM $CaCl_2$, 10mM Potassium MES) (2- N-morpholine ethane sulphonate) and incubated at 0° for 10 minutes as 10mM Potassium MES) and incubated at 0° for 10 minutes as indicated in Fig. 2, each (2ul) of the ligation reaction mixes was then incubated in 200ul of the calcium treated bacteria for 30 min. at 0°. The transformation mix was heated briefly (90 seconds) to 42° and 4ml aliquots of melted top agar (Luria Broth + 0.9% Agar) containing 100 ul of a saturated culture of JM101, 10ul of 0.1M IPTG (isopropyl-thiogalactoside) and 100ul of 2% X-gal (5-bromo-4-chloro-3-indolyl-β-D-galactoside) were added and the mixture spread over the surface of 0.9% Agar plate containing Luria Broth. The plates were incubated overnight at 37° to allow phage plaques to develop. Because the M13 vector carries the E. coli β-galactoside, all plaques from non-recombinant phages will be blue because of the IPTG and X-gal included in the plating mix (a plaque is a clearing in the lawn of bacteria on the plate caused by a bacteriophage clone). If, however, the β-galactosidase gene is interrupted by insertion of a piece of foreign DNA, then β-galactosidase will not be expressed and the phage plaques will be colorless (or white). Each transformation yielded between 20 and 130 white plaques. To identify the desired phage clones carrying TCGF DNA sequences, an imprint of each plate was prepared by carefully placing an 85mm circular nitrocellulose filter on the agar surface. The filter was carefully lifted from the plate after marking its orientation relative to the surface of the plate. This filter, which picked up small amounts of phage particles at the location of each plaque, was rinsed briefly in 0.5M Tris, pH 7.5, 1.5M NaCl, then in 1 x SSC and finally was baked in vacuo at 80° for 2 hours. The filter was hybridized to the gTCGF cDNA insert (labeled with $^{32}$p by nick-translation (Rigby et al. J. Mol. Biol. 113, 237) using the standard hybridization procedure with incubation at 68°. Two plaques that hybridized to the labeled probe were picked from each of the original size classes of degraded cDNA fragments for sequencing. Each positive plaque was picked with a pasteur pipet and transferred into 2 ml of JM101 which had been freshly diluted 100 fold with SOBM broth. Each infected culture was grown at 37° for 4.5-6 hours with vigorous shaking. The bacteria were then removed from each culture by centrifugation (5 min. in the microfuge). The phage-containing supernatants were collected, 150 ul of 20% polyethylene glycol and 2.5 M NaCl was added and the phage allowed to precipitate for 10 min. at room Temp. Each precipitate was collected (5 min. in microfuge) being careful to remove all of the supernatant. The pellets were each resuspended in 100ul of TE, 0.3M sodium acetate, extracted with phenol and the DNA was ethanol precipitated from the aqueous phase. Each DNA pellet was rinsed with 95% ethanol, dried in vacuo and resuspended in 30ul of TE buffer. The length of each of the five degraded fragments within the resulting collection of single stranded templates is shown in Fig. 3 with the solid line portion representing the stretch actually sequenced and the dotted line portion not sequenced.

In method a-2)-restriction cleavage-templates for dideoxy sequencing were generated by cleavage of 10ug of the pBR322-gTCGF with combinations of restriction enzymes (indicated in Fig. 3), gel purifying the fragments and ligating them to the appropriate M13 vectors as described above for the deletion series (method a-1). These fragments included the 250 base pair Pst I fragment at the 3' end of the clone (cloned into Pst I linearized mp 9); the ca 300 base pair fragment at the 5' end of the gTCGF sequence generated by cleavage with Rsa I and Pst I (cloned into mp 9 cut with Sma I and Pst I); the ca 350 base pair fragment generated by cleavage with Rsa and Xba I (cloned into mp 11 cut with Xba I and Sma I); the ca 400 base pair fragment generated by cleavage of the cDNA with Xba I and Pst I (cloned into M13 vector mp 11 cut with

Xba I and Pst I) and the ca 200 base pair fragment generated by digestion with Stu I and Pst I (cloned into mp 9 cut with Sma I and Pst I). In each case several white plaques were picked and single stranded templates were prepared for Dideoxy Sequencing as described in Method A), above. The stretches that were sequenced from each clone are represented in Figure 3. b) By the Maxam-Gilbert method as described in Methods in Enzymology 65, 499 (1980). The sequence strategy is represented in Fig. 4 in which the non-labelled ends of excised fragments are in the direction of the arrows. As represented in Fig. 4, the cloned DNA (pBR322-gTCGF) was linearized with Xba I and the 5' end labelled by the polynucleotide kinase reaction ($r^{32}$P-ATP and T4 polynucleotide kinase). The labelled DNA was then cut with EcoRI which generated 2 fragments (shown Fig. 4). Other linearized segments were obtained from the cloned DNA by cutting with Pst I and using the cordysepin reaction [$^{32}$-P-cordysepin and terminal transferase described in Tn, C.-P.D. and Cohen, S.N., Gene 10, 177-183(1980)] to label the 3' ends of the segments which were then treated with Xba I and Pvu II to generate 4 fragments of which 3 were isolated (shown Fig. 4). In addition, the cloned DNA was linearized with Pvu II followed by labelling by the polynucleotide kinase reaction and cleavage of the resulting segment with Bgl I to generate 2 fragments (shown Fig. 4). All fragments in each preparation were resolved from one another by electrophoresis through 1.0% Agarose gel, and isolated from the gel by the glass powder protocol (see below). All fragments were then sequenced by the standard chemical degradation method, and the length sequenced is shown in Fig. 4 by solid lines.

STEP M: Transfer Vector for Expression in Animal Cells

By the procedure represented in Fig. 5, 10 μg of the cloning plasmid pbR322-gTCGF was digested with 20 units of the endonuclease Pvu I for one hour at 37° in 100 μl of 50 mM tris-HCl, ph 7.8, 10mM $MgCl_2$, 10mM 2-mercaptoethanol and 100 mM NaCl. The resulting linearized DNA (5 μg) was treated with 600 units of Exonuclease III in 100 μl of 50 mM Tris; HCl pH 8, 10mM of 2-mercaptoethanol and 5 mM of $MgCl_2$. Aliquots of 15 μl were taken after 30, 90 and 120 seconds and added to 300 μl of 0.5 M NaCl and 20 mM EDTA. The combined ethanol precipitated DNA aliquots were then incubated with 160 μl of 30mM sodium acetate, pH 4.6, 200 mM NaCl, 1mM $ZnSO_4$ and 40 units of S1 nuclease. The reaction was stopped after 30 minutes at 30° by the addition of 40 μl of 0.5M Tris-HCl, pH 8.0 and 1M of NaCl. The ethanol precipitated and collected DNA was then incubated in 100 μl of 10mM Tris-HCl, pH 7.5, 100 uM each of dATP, dCTP, dGTP and TTP, 1mM dithiothreitol, 10mM $MgCl_2$ and 5 units of Klenow fragment of DNA polymerase I for 15 minutes at 37°. The reaction was stopped by addition of NaCl (to 0.2M final concentration) and extraction with phenol/chloroform. The resulting blunt ended DNA was collected by ethanol precipitation and ligated to Pst I adaptors prepared by treating 300 pmole of the 10 mer with T4 polynucleotide kinase and 1 mM ATP, stopping the reaction by extraction with phenol/chloroform, adding 300 pmole of the 15 mer, and ethanol precipitating. The 15 mer plus the 10 mer were resuspended in 30 μl of 10mM Tris, pH 7.5, 1 mM of EDTA and 0.2 M NaCl and annealed at 15° for 2 hours. The adaptor structure is as follows:

```
5'  C-T-A-G-A-G-G-C-C-T-C-T-G-C-A-OH  3'

3'  G-A T-C-T-C-C-G-G-A               5'
```

The adaptor (50 pmole) was ligated to the degraded and blunted DNA in 30 µl of 25 mM Tris HCl, pH 7.5, 10mM $MgCl_2$, 10 mM DTT, 100 µM ATP and 0.1 unit of T4 ligase at 15° for 90 minutes, and the reaction stopped by heating at 68° for 15 minutes. The "Pst I adapted" DNA was then digested with Pst I (20 units) for 4 hours at 37° after adding NaCl to 66mM (final volume 31 ul). The reaction mixture (without extraction or precipitation of DNA content) was then electrophoresed through a 1% Tris acetate gel and the region between 500 and 600 nucleotides excised from the gel and the DNA content isolated by the glass powder protocol (see below). This gTCGF code-containing DNA segment was then integrated into the sites created by Pst I cleavage of the plasmid pCVSVL which, as evident from Fig. 5, includes the adenovirus major late promoter (AdMLP), a 5' splice site (5'SS); a 3' splice site from a mouse immunoglobulin gene (3'SS); the polyadenylation site from the SV40 early region (pA); and a duplicated SV40 origin of replication (SV40 ori). Selectable genes in the plasmid are tetracycline resistance ($Tet^R$) and mouse dihydrofolate reductase (DHFR). This plasmid is particularly suitable for expression of genes under the control of its promotor in monkey COS-7 cells. Thus, plasmid pCVSVL (20 µg) was digested with 30 units of Pst I in 100 µl of 50 mM Tris-HCl, pH 7.8, 10mM of $MgCl_2$, 10 mM 2-mercaptoethanol and 50mM NaCl., and the linearized plasmid (50 ng) after extraction with phenol and precipitation with ethanol was incubated overnight at 16° with 25 ng of the gTCGF gene segment in 20 µl of 25 mM Tris HCl, pH 7.8, 10 mM $MgCl_2$, 10 mM 2-mercaptoethanol and 50 mM NaCl and linearised plasmid (50 ng) after extraction with phenol and precipitation with ethanol was incubated overnight at 16° with 25 ng of the gTCGF gene segment in 20 ul of 25 mM Tris HCl, pH 7.8, 10 mM $MgCl_2$, 10 mM DTT, 1m MATP and 0.1 unit of T4 ligase. The resulting transformation mixture was spread onto nitrocellulose disks on 4 different agar plates (10 µl/ml tetracycline each). After overnight incubation, two replicas of each filter were prepared according to the procedure described

above in Steps G and H. One of each replica was hydridized to the 5' oligonucleotide probe used in Step I, above, and the other replica hydridized with the 3' probe (Step I, above), as described in Step I, above. Following the procedure of Step J, above, it was found that most colonies hydridized with both probes and six colonies were selected and cultured overnight in 2 µl of L-broth. The transfer vectors within the cultured colonies was then liberated using the standard rapid DNA preparation method as described in Step K, above. Each rapid preparation DNA was analyzed by digestion with Pst I and one clone which had a Pst I insert of about 600 nucleotide was lysed and CsCl banded by the following procedure:

1. Grow 1 liter of plasmid containing bacteria o/n in L-broth (10-15 ug/ml Tet)
2. Harvest cells-4000 rpm in Beckman R-6B. 10 minutes.
3. Wash cells in 250 ml cold TE (10mM Tris-HCl, in 1 mM EDTA, pH 8).
4. Resuspend cells in 15-20 ml of 25% sucrose, 0.05M Tris-Hcl, pH 7.5 at 0°C.
5. Add 1/10 volume 1.5-2.0 ml of lysozyme (10 mg/ml in sucrose solution). Incubate on ice 10 min.
6. Add 1/5 volume (3-4 ml) 0.5M EDTA, pH 8. Incubate on ice for 5 min.
7. Dilute suspension 1:1 with cold triton lysis solution. Incubate on ice 10 min.

   Triton Solution: 0.1% triton X-100<br>60mM EDTA<br>50mM Tris-HCl pH 8
8. Spin 18K in Sorval SS34 rotor for 30 minutes.
9. Decant the supernatant. Add 0.95 g/ml CsCl and 1/10 volume 10 mg/ml ethridium bromide-Refractive index should be between 1.390 and 1.396.
10. Spin at 45K in the 50.2 Ti Beckman rotor for 2 days.

11. Using a longwave UV light to illuminate the tube, carefully withdraw the plasmid DNA (lower band) from the gradient. Extract the ethidium bromide from the DNA by extracting with an equal volume of TE saturated butanol three times. Finally, precipitate the DNA by diluting it 1:3 with water and adding 2.5 volumes of ethanol (-20°C for 3-4 hours). Collect the precipitate by centrifugation (4000 RPM, Beckman R6B, 15 min). Resuspend in 200 ul $H_2O$.
12. To prepare twice banded plasmid DNA, take the first band and transfer it directly into a vti65 tube. Fill the tube with CsCl solution (refractive index 1.393). Spin 18 hours at 45,000. Withdraw band and continue as in 11) above.

The CsCl banded DNA was then cleaved with Pst I and the fragments labelled with $^{32}P$-cordysepin and terminal deoxynucleotidyl transferase by the procedure described in Step L, above. The labeled fragments were cleaved with Xba I and the resulting 2 fragments isolated from an agarose gel and sequenced by the Maxam-Gilbert method. Sequencing of the 5' fragment showed that the Pst I adaptor had been fused to the sequence:

ACTCACAG. . .

in the 5' non-coding region of the gTCGF gene-containing insert and that the artifact DNA section plus 19 nucleotides of the 5' untranslated region had been deleted. The correct orientation of the gTCGF gene sequence in the transfer vector was confirmed by standard measurement of fragments from Rsa I and Xbo I restriction cleavage. The transfer vector conforming to the correct structure was designated pCVSVL-gTCGF.

STEP N: Expression of pCVSVL-gTCGF in Animal Cells

Following the DEAE-Dextran method described by Sompayrac et al., Proc. Nat. Acad. Sci. USA 78, 7578-7578 (1981), 10 cm dishes of subconfluent COS-7 monkey cells were transfected with 8 μg of pCVSVL-gTCGF in 4 ml. of DEAE-Dextran solution for 10 hours. After rinsing with DME (purchased from Gibco) the cells were treated with DME containing 0.1 mM choroquin, as more particularly described by Luthman et al., Nuc. Acids Res. 11, 1295-1308 (1983). After 2.5 hr at 37°, this medium was replaced with DME +10% heat inactivated fetal calf serum and the dishes were incubated for 48 hours and the cells were fed 4 ml of fresh DME containing 10% heat inactivated fetal calf serum. After an additional 24 hours, the conditioned medium (8 ml) was collected and the cells were scraped into 5 ml of cold phosphate buffered saline. The cells were then collected by centrifugation (15 minutes in the Beckman model 6B) and the conditioned medium was assayed using the TCGF dependent mouse cell line CTLL-2 (described by Stull et al., J. Immunol. 126, 1680-1683 (1981)). In this assay, $10^4$ CTLL-2 cells are incubated for 24 hours in microtiter wells in 2-fold serial dilutions of the sample to be assayed. The mouse cells are then pulsed for 4 hours with 0.5 μC/well $H^3$-Thymidine. The cells are collected on glass fiber paper using an automatic harvesting device and the radioactivity in the cells determined. One unit of TCGF activity/ml is defined as the dilution which gives 50% of maximal incorporation using $10^4$ cells in 150 ul of RPMI-1640 plus 5.0% human type AB serum. By this Assay it was found that the transfection of monkey COS-7 cells with the transfer vector pCVSVL-gTCGF resulted in 250 units/ml of TCGF activity. Isolation of the active expression product from the monkey COS-7 cell medium and sequencing of this protein confirms that gTCGF conforms to the structure of polypeptide having sequentially the amino acids 21-153, inclusive, as set forth in Table A, above, with normal N- and free acid C-termini.

## Example A

Preparation of plasmid pEVPL: The constructing of the plasmid pEVPL is represented in Fig. 8. Employed as a starting material was the plasmid pKC30cII which is described by Shimatake and Rosenberg, Nature, 292(1981), pages 128-132 and also discussed by Maniatis et al, Molecular Cloning, A Laboratory Manual, Cold Spring Harbor Laboratory, 1982, Chapt 12, pages 404-433, the disclosure of both said publications being incorporated herein by reference as to the structure and properties of pKC30cII to the extent not described herein. The plasmid includes sequentially downstream from it $P_L$ promoter-operator an anti-termination recognition site (Nut L), the N gene region (N/2), an anti-termination recognition site (Nut R), the transcription termination signal (tRl), the cII gene region comprising its ribosome binding site sequence (RBSS) and coding sequence preceded by its ATG translation initiator, half of the O gene region (O/2) and the transcription termination signal (tL), all variously depicted in Fig. 8. The plasmid pKC30cII (10 µg) is first digested with 20 units of the endonuclease BamHI in 100 µl of 25 mM Tris, pH 8, 10 mM $MgCl_2$, 10 mM 2-mercaptoethanol, 50 mM NaCl and 100 µg/ml BSA for 1 hour at 37° to obtain after extraction (phenol/chloroform and twice with chloroform and ethanol precipitation) the linearized plasmid (shown Fig. 8). The objective was then to cut back from the BamHI terminus approximately 1000 b.p. to reach the ATG translation initiation codon for the protein coded for by the cII coding sequence. For this purpose 10 µg of the linearized plasmid was incubated with 1200 units of Exonuclease III in a 200 µl reaction (composed of 50 mM Tris, pH 8, 10 mM 2-mercaptoethanol and 5 mM $MgCl_2$) at 30°C. Aliquots (of 65 µl) were removed at 6, 7 and 8 minutes on the expectation that the condition of treatment removed about 150 bases/ minute. The aliquots were combined in a tube containing 100 µl of 40 mM EDTA and 1M NaCl, and ethanol precipitated.

The resulting combined 3' degraded DNA segments were then incubated with 80 units of Nuclease S1 in 320 ul of S1 buffer (composed of 30 mM NaAc, pH 4.6, 200 mM NaCl and 1 mM $ZnSO_4$) for 30 minutes at 30°. in order to substantially remove the extensive 5' overhangs remaining from the Exo III treatment. The action of the Nuclease S1 was stopped by the addition of 40 µl of 0.5 M Tris-HCl, pH 8.0, and 1 M NaCl and the resulting DNA collected by ethanol precipitation after extraction with phenol/chloroform and chloroform. The resulting DNA segments were then blunt ended by incubating in 100 µl of 10mM Tris HCl pH 7.5, 100uM each dATP, dCTP, dGTP, and dTTP, 1mM dithiothreitol 10mM $MgCL_2$ and 5 units of the Klenow fragment of DNA polymerase I for 15 min. at 37°. The reaction was stopped by addition of NaCl to 0.2M, and the mix was extracted with phenol/chloroform and several times with chloroform and the DNA collected by ethanol precipitation. The above reactions produce from each aliquot a multiplicity of different length segments represented by the three segments shown in Fig. 8. The subsequent treatments described below are represented in Fig. 8 only by the single desired segment in order to show more detail. The resulting blunt ended DNA segments were ligated to a unique BamHI adaptor prepared from a 10 mer and a 14 mer having the sequence:

5' GTACCTATGG 3'

3' CATGGATACCCTAG 5' ,

said adaptor being phosphorylated as described for Bam HI adaptors on page 5 of the M13 Cloning Dideoxy Sequencing Manual, *supra*. In addition to providing a BamHI cohesive end, this adaptor begins with 5 of the 6 nucleotides necessary for a KpnI site and will create said site when ligated to the ATG codon of the cII sequence. The adaptor (30p mole) was therefore ligated to the blunt end DNA by incubating in 60ul of 25 mM Tris HCl pH 7.5, 10mM $MgCl_2$, 10mM dithiothreitol, 100uM ATP and 0.2 units of T4 ligase at 15° for 90 minutes. The reaction was stopped by heating to 68° for 15 min. The resulting DNA segments with their unique Kpn-BamHI adaptors (Kpn-BA) are represented in Fig. 8. The DNA from the adaptor ligation was diluted to 100ul and NaCl added to a final concentration of 0.1M. This reaction mix was incubated with the endonuclease EcoRI (20 units) for 60 min. at 37°. The reaction mix was extracted with phenol/chloroform, chloroform, ethanol precipitated and electrophoresed through a 1% Tris acetate agarose gel. The gel purified DNA segments in the size class of 2500 to 3000 b.p. was isolated from the gel using the flass powder protocol.

A pBR322 plasmid segment was prepared by digesting pBR322 DNA (10 µg) sequentially with the endonuclease BamHI 1 hr. at 37° in 100 µl (10mM TrisHCl, pH 7.5 and 50 mM NaCl, 1 mM DTT), then raising the NaCl concentration to 0.1M and cutting with the endonuclease EcoRI (20 units for 1 hr. at 37°). The reaction was stopped by extraction with phenol/chloroform and extracted twice with chloroform and ethanol precipitated. The 3985 base pair vector fragment was purified by electrophoresis through a Tris acetate gel followed by glass powder isolation of the DNA (see below).

The above prepared pBR322 plasmid segment (50 ng) was then ligated with 25ng of the selected gel purified DNA segments (2500-3000 b.p.) in a reaction mix (25 ul) containing 25 mM Tris-HCl, pH 7.5, 10mM $MgCl_2$, 10mM DTT, 100uM ATP, and 0.1 units of T4 ligase (at 15° overnight). This reaction was used to transform *E. coli* W3110 lambda Y139 which carries a temperature sensitive lambda repressor protein (on a lambda lysogen in the bacterial chromosome) which can be used to shut off the $P_L$ promoter. The transformation was performed by incubating 10ul of the ligation with 200ul of transformation competent bacteria ($CaCl_2$ shocked) for 30 minutes on ice, 2 minutes @ 37°, then 30 minutes at 30° after diluting to 1 ml with L broth. The transformed bacteria were spread directly onto 4 nitrocellulose filters on L-broth containing agar plates having 25 ng/ml ampicillin. After incubation overnight at 30°, 2 replicas were prepared from each filter. These replicas were amplified by incubation overnight on L Broth agar plates containing 100 mg/ml chloramphenicol. The filters were prepared for colony hybridization by standard treatment with base (Step H above). One set of filters was probed overnight at room temperature with 100,000 CPM/ml of the 14 mer from the adaptor which had been $^{32}P$-labelled. The other set of filters were hybridized to a junction oligonucleotide which spans the junction of the adaptor to the ATG of the cII sequence and which is a 14 mer having the sequence:

ACATATGGTACCTA

wherein the ACATATG is the sequence leading up to the first ATG of the cII sequence and GTACCTA is the start of the BamHI adaptor. Several colonies which hybridized to both of these probes were used to prepare plasmid DNA (CsCl density gradient protocol, Step M, Example 1). The DNAs were tested for the presence of a single BamHI site and a single Kpn I site. One such plasmid was further checked by Maxam-Gilbert sequencing (labeled by the T4 kinase reaction at the BamHI site), and found to have the desired sequence. This plasmid was designated pEVPL.

## Example B

## Expression of gTCGF in pEVPL-gTCGF in 35S-Met labelled E. Coli W3110 Lambda Y139

E. Coli W3110 λ139 (10 ml) which has been transformed with pEVPL-gTCGF is grown overnight at 30°. in M63 medium and 5% L-Broth in the presence of 25 μg/ml of ampicillin.

The temperature of the cell mass is then raised to 10° for 10 minutes, cooled to 37° and the cells labelled with $^{35}$S-methionine (10 μCi/ml) for 2-3 minutes, followed by chasing with 300 μl of cold methionine (0.5%) per each 5 ml of culture. Aliquots (0.5-1.0 ml) are collected at several different time intervals after labelling, e.g. 10, 30, 60, 120 and 150 minutes. Each aliquot sample is prepared by collecting the cells (5 minute spin in microfuge) and resuspending in 100 μl SDS gel sample buffer (25 mM Tris-HCl, ph 6.8, 2% SDS, 20% glycerol, 0.002% bromophenol blue and 100 mM 2-mercaptoethanol). The proteins from the samples are then fractionated on a 15% polyacrylamide SDS gel in accord with the method described by Laemmli, Nature 227, page 680 (1970). In this manner it was confirmed that expression of pEVPL-gTCGF in E. Coli W3110 lambda Y139 produces good quantities of a protein product having the ca 15,000 Dalton molecular weight of TCGF. In this Example B the M63 medium is prepared by mixing 100 ml of a sterilized 1:5 water dilution of a 1 liter aqueous mixture of 15 g. $KH_2PO_4$, 35 g. $K_2HPO_4$, 10 g. $(NH_4)_2SO_4$ and 2.5 ml $FeSO_4$ (1 mg/ml) with sterilized mixture of 10 ml of 20% glucose and 1 ml 1M $MgSO_4$.

While the invention has been described with reference to certain particular embodiments, it will be evident that numerous modifications may be made as a result of and within the scope of the invention, and in the practicing thereof, including but in no way limited to, modifications in vectors, transfer vectors, probes, cell systems and microorganisms for expression, and in procedures for construction and analysis, all as evident to those skilled in the art. Similarly, in view of the variability of the genetic code, the cDNA sequence coding for gTCGF may be modified, e.g. by the known process of site specific mutagenesis, to produce a different sequence of nucleotides coding for the same protein.

In the glass powder protocol referred to above,

**the desired fragments are excised from the ultra violet illuminated agarose gel. The agarose slices are dissolved in NaI solution (90% NaI, 1.5% $Na_2SO_3$) using 1 ml of solution per gram of gel slice. The DNA is next adsorbed to 5ul of a 50% slurry of acid washed glass powder (325 mesh silica) (5ul will bind up to 20ug of DNA) for 15 min. on ice. The glass powder is collected by centrifugation (1 min in the microfuge) and washed 2 times with 100ul of NaI solution. To remove traces of NaI, the glass powder pellet is washed 2 times with cold 50% ethanol containing 0.1 M NaCl, 10mM Tris-HCl, pH 7.5, 1mM EDTA. Finally the DNA is eluted from the glass by incubation at 37° with 10ul of 10mM Tris-HCl, pH 7.5, 1mM EDTA.'**

CLAIMS

1. A transfer vector comprising a DNA sequence coding for gTCGF.

2. A transfer vector according to claim 1, comprising a recombinant operon comprising a DNA sequence coding for gTCGF.

3. A transfer vector according to claim 2, comprising a recombinant operon comprising a DNA sequence coding for gTCGF, the ATG codon upstream from and in reading phase with said sequence and one or more translation termination signals immediately following said sequence downstream.

4. A transfer vector according to claims 1, 2 or 3, in which said DNA sequence includes a base sequence corresponding to amino acids 1 to 153 of Table A.

5. A transfer vector according to claims 1, 2 or 3, in which said DNA sequence includes a base sequence corresponding to amino acids 21 to 153 of Table A.

6. A transfer vector according to claim 5, in which said base sequence is immediately preceded upstream by ATG.

7. A prokaryotic or eukaryotic host transformed with a transfer vector according to any one of the preceding claims.

8. A method of producing gTCGF comprising culturing a prokaryotic or eukaryotic host according to claim 7, and recovering the gTCGF so produced.

9. gTCGF, whenever produced by a process according to claim 8.

10. A protein comprising the amino acid sequence of amino acids 1 to 153 of Table A.

11. A protein comprising the amino acid sequence of amino acids 21 to 153 of Table A.

12. A protein comprising the amino acid sequence of amino acids 21 to 153 of Table A in which amino acid 21 of Table A is immediately preceded by Met.

13. A mixture of the proteins according to claims 11 and 12.

14. A pharmaceutical composition comprising a therapeutically effective amount of a protein according to any one of claims 9 to 12, in association with a pharmaceutically effective diluent or carrier.

5'
AAAAA 3' (mRNA)
Reverse Transcriptase
5'
AAAAA 3' (mRNA/cDNA)
3'
TTTT
DNA Polymerase
E. Coli ligase and
E. Coli RNAse H.
5'
(Loopback cDNA)
3'
SI nuclease
3'
5'
(duplex cDNA)
5'
3'
Terminal Transferase
("C" tailing)
3'CCCC
5'
CCCC3'
cDNA(gTCGF)insert
Mix
and
anneal
GGGG
CCCC
CCCC
GGGG
pBR322-cDNA
(pBR322-gTCGF)
Pvu I
EcoRI
BamHI
GGGG3'
5'
3'GGGG
5'
Pvu I
pBR322 cleaved
with Pst I and
"G" tailed

FIG. 1

Pst I
Pst I
Pst I
EcoRI
Bam HI
Pvu I
Digest
Pvu I
EcoRI
BamHI
Pst I
cDNA
gTCGF
insert
pBR322-gTCGF
ca4300b.p.
126b.p.
Pst I
Pst I
1) Exo III for various times
2) Nuclease S1
3) Klenow
M13mp9
EcoRI
Bam HI
Pst I
Hind III
30b.p.
Bam HI and
Pst I
Digestion
1) Mix
2) Ligate
3) Transform E. Coli
1) BamHI adaptors(BA)
2) T4 Ligase
3) Pst I digestion
BA

FIG. 2

- 3/8 -

b.p.

0 100 200 300 400 500 600 700 800 900 1000

5' 3'

Artifact

gTCGF Coding region

PstI RsaI XbaI StuI PstI PvuII PstI

BA BA BA BA BA

Fragments from fig. 2 with BamHI adaptors (BA)

FIG. 3

0
100
200
300
400
500
600
700
800
900
1000
5'
3'
Artifact
gTCGF Coding region
PstI
RsaI
XbaI
StuI
PstI
PvuII
PstI
To EcoRI
To EcoRI
PstI to XbaI
PstI to PvuII
PstI to XbaI
to BglI
to BglI

FIG. 4

pBR322-gTCGF
PvuI Digest
Total cDNA-ca1000b.p.
PstI
ca800b.p.
5' TAGC
GC 3'
gTCGF Sequence-133b.p.
Artifact
ca 3500b.p.
PstI
Untranslated region
1) Exonuclease III
2) Nuclease SI with Klenow fragment
3) PstI adaptors
Signal region of 60b.p.
gTCGF Sequence
PstI
PstI Digest
Remainder of untranslated region ca 28b.p. with PstI adaptors
ca 606 b.p.
gTCGF Sequence
5'
3'
Signal region of 60b.p.
pCVSVL
PstI Digest
Mix
DNA Ligase
XhoI
XhoI
XhoI
5'SS
EcoRI
SV40ori
Hind III
AdMLP
3'SS
Signal region
pCVSVL-gTCGF
Tet R from pBR322
cDNA Insert
gTCGF
PstI reestablished
DHFR
pA

FIG.5

pBR322-gTCGF
Digest
Pst I
ca 760b.p.
ca 240b.p.
Reading direction of gTCGF coding sequence
gTCGF Coding sequence
Pst I
BamHI
M13 mp9
Digest
Pst I
12b.p.
BamHI
Mix
1) Ligate
2) Transform E. Coli
3) Select plaques
gTCGF ca 760b.p.
dsM13-gTCGF
Pst I
Bam HI
Process to single stranded DNA
(Pst I)
ca 760b.p. Region
ssM13-gTCGF
(Pst I)
(BamHI)
21-Ala Primer
(5' 17mer)
Primer (17 bases)
(Pst I)
(Bam HI)
(Pst I)
Region of ca 760b.p.
ds primed ssM13-gTCGF
Klenow
(Pst I)
ds gTCGF Coding sequence
Bam HI
Pst I
Partially ds M13-gTCGF
1) Nuclease SI
2) Klenow
Pst I
12b.p.
Total residue of gTCGF insert
BamHI
BamHI Digest
BamHI cohesive end
5' G
3' GATCC
Pst I
gTCGF Coding sequence
3'
Blunt end
gTCGF Segment BE

FIG. 6

Lambda $P_L$ Promoter-operator
EcoRI
cII Ribosome binding site sequence
pEVPL
KpnI
BamHI
KpnI Digest
EcoRI
Lambda $P_L$ Promoter-operator
cII Ribosome sequence
5'C
CATGG 3'
ATGGTAC 3'
TAC 5'
BamHI
1) Klenow
2) BamHI digest
Lambda $P_L$ Promoter-operator
cII Ribosome binding site sequence
5'GATCC
3'G
ATG 3'
TAC 5'
EcoRI
PstI
GTGF Coding sequence
Ligate
Mix
5'G
3'GATCC
gTCGF Segment BE
Lambda $P_L$ Promoter-operator
EcoRI
cII Ribosome binding site sequence
GTA
CAT
pEVPL-gTCGF
gTCGF Coding sequence
PstI
BamHI

FIG. 7

pKC30cII
o/2
cII
N/2
tL
PL
Hind III
BamH I
EcoR I

BamH I digest

tL cII tRI N/2 PL EcoR I
o/2 NutR NutL Hind III

1) Exonuclease III
2) Nuclease SI
3) Klenow (blunt)

o/2 cII PL EcoR I
Hind III
cII PL EcoR I
Hind III
tR2 PL
EcoR I
Hind III

BamH I
adaptors (BA)
Ligate

BA cII PL EcoR I BA
Hind III

EcoR I digest

pBR322
EcoR I
BamH I
AmpR
TetR

1) BamH I digest
2) EcoR I digest

Mix
Ligate

Kpn I
3' GGTATCCATGGTA
GATCCCATAGGTACCAT
BamH I
adaptor
cII RBSS
tR N/2 PL
NutR NutL Hind III

pEVPL
KpnI
CCATGGTA
GGTACCAT
cII RBSS
BamH I
tRI
N/2
PL
EcoR I
TetR
AmpR

FIG. 8

European Patent Office

# EUROPEAN SEARCH REPORT

0141779

Application number

EP 84 81 0471

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, Vol. 100, No. 7, February 13, 1984, page 145, Abstract nr. 46294k, COLUMBUS, OHIO, (US). L. YUAN:"Cloning of double stranded cDNA from major and minor components of mRNA". & NSC Symp. Ser. 1982, 4, 108-28 --- | 1-3, 7-9 | C 12 N 15/00<br>C 12 P 21/02<br>C 07 K 7/10<br>A 61 K 37/02 |
| Y | THE JOURNAL OF BIOLOGICAL CHEMISTRY, Vol. 257, No. 4, February 25, 1982, pages 1587-1590, (US). Y. LIN et al.:"Synthesis of biologically active interleukin 2 by XENOPUS oocytes in response to poly(A)-RNA from a gibbon T-cell line". * Whole document * --- | 1,2, 7-9 | TECHNICAL FIELDS SEARCHED (Int. Cl.4)<br>C 12 N<br>A 61 K |
| Y | EP-A-0 088 195 (TAKEDA CHEMICAL INDUSTRIES, Ltd.) * Claims 1-10 * --- | 1,2, 7-9 | |

-/-

The present search report has been drawn up for all claims

| Place of search | Date of completion of the search | Examiner |
|---|---|---|
| THE HAGUE | 10-01-1985 | DELANGHE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0141779
Application number

EP 84 81 0471

page 2

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| A | NATURE, Vol. 302, March 24, 1983, pages 305-310 TADATSUGU TANIGUCHI et al.:"Structure and expression of a cloned cDNA for human interleukin-2". * Whole document * --- | 1,2, 7-9 | |
| A | EP-A-0 089 062 (AJINOMOTO CO., INC.) * Claims 1-9 * ----- | 1,14 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 10-01-1985 | DELANGHE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82